# EUROPEAN PATENT APPLICATION

(11) **EP 2 073 010 A1**
(43) Date of publication of application: **24.06.2009**
(21) Application number: 07150072.2
(22) Date of filing: 17.12.2007
(51) Int. Cl.: G01N 33/574

(54) **Means for selecting a breast cancer treatment**

(71) Applicant: Atlas Antibodies AB, 106 91 Stockholm (SE)
(72) Inventor: Uhlén, Mathias, SE-182 79, Stocksund (SE); Pontén, Fredrik, SE-752 37, Uppsala (SE); Jirström, Karin, SE-216 18, Limhamn (SE)
(74) Representative: Engdahl, Stefan

(57) **Abstract**

The present invention provides a new method and means for determining whether a mammalian subject having a breast cancer is likely to benefit from an endocrine treatment. The method comprise the steps of: providing a sample earlier obtained from said subject; evaluating the amount of CRABP2 protein present in at least part of said sample, and determining a sample value corresponding to said amount; comparing the obtained sample value with a reference value; and, if said sample value is higher than said reference value, concluding that the subject is likely to benefit from an endocrine treatment.

SEQUENCE LISTING

<110>  Atlas Antibodies AB

<120>  Means for selecting a breast cancer treatment

<130>  21021209

<160>  3

<170>  PatentIn version 3.3

<210>  1
<211>  94
<212>  PRT
<213>  Homo sapiens

<400>  1

Ile Lys Gln Glu Gly Asp Thr Phe Tyr Ile Lys Thr Ser Thr Thr Val
1               5                   10                  15

Arg Thr Thr Glu Ile Asn Phe Lys Val Gly Glu Glu Phe Glu Glu Gln
            20                  25                  30

Thr Val Asp Gly Arg Pro Cys Lys Ser Leu Val Lys Trp Glu Ser Glu
        35                  40                  45

Asn Lys Met Val Cys Glu Gln Lys Leu Leu Lys Gly Glu Gly Pro Lys
    50                  55                  60

Thr Ser Trp Thr Arg Glu Leu Thr Asn Asp Gly Glu Leu Ile Leu Thr
65                  70                  75                  80

Met Thr Ala Asp Asp Val Val Cys Thr Arg Val Tyr Val Arg
                85                  90

<210>  2
<211>  138
<212>  PRT
<213>  Homo sapiens

<400>  2

Met Pro Asn Phe Ser Gly Asn Trp Lys Ile Ile Arg Ser Glu Asn Phe
1               5                   10                  15

Glu Glu Leu Leu Lys Val Leu Gly Val Asn Val Met Leu Arg Lys Ile
            20                  25                  30

Ala Val Ala Ala Ala Ser Lys Pro Ala Val Glu Ile Lys Gln Glu Gly
        35                  40                  45

Asp Thr Phe Tyr Ile Lys Thr Ser Thr Thr Val Arg Thr Thr Glu Ile
    50                  55                  60

Asn Phe Lys Val Gly Glu Glu Phe Glu Glu Gln Thr Val Asp Gly Arg
65                  70                  75                  80

Pro Cys Lys Ser Leu Val Lys Trp Glu Ser Glu Asn Lys Met Val Cys
                85                  90                  95

Glu Gln Lys Leu Leu Lys Gly Glu Gly Pro Lys Thr Ser Trp Thr Arg
            100                 105                 110

Glu Leu Thr Asn Asp Gly Glu Leu Ile Leu Thr Met Thr Ala Asp Asp
        115                 120                 125

Val Val Cys Thr Arg Val Tyr Val Arg Glu
    130                 135

<210>  3
<211>  783
<212>  DNA
<213>  Homo sapiens

<400>  3
ccagagaagc ctggacccac acgcgggcta gatccagaga cagtgtccag tgctccagcc      60

taggagtcta cggggaccgc ctcccgcgcc gccaccatgc ccaacttctc tggcaactgg     120

aaaatcatcc gatcggaaaa cttcgaggaa ttgctcaaag tgctgggggt gaatgtgatg     180

ctgaggaaga ttgctgtggc tgcagcgtcc aagccagcag tggagatcaa acaggaggga     240

gacactttct acatcaaaac ctccaccacc gtgcgcacca cagagattaa cttcaaggtt     300

ggggaggagt ttgaggagca gactgtggat gggaggccct gtaagagcct ggtgaaatgg     360

gagagtgaga ataaaatggt ctgtgagcag aagctcctga agggagaggg ccccaagacc     420

tcgtggacca gagaactgac caacgatggg gaactgatcc tgaccatgac ggcggatgac     480

gttgtgtgca ccagggtcta cgtccgagag tgagtggcca caggtagaac cgcggccgaa     540

gcccaccact ggccatgctc accgccctgc ttcactgccc cctccgtccc accccctcct     600

tctaggatag cgctcccctt accccagtca cttctggggg tcactgggat gcctcttgca     660

gggtcttgct ttctttgacc tcttctctcc tcccctacac caacaaagag gaatggctgc     720

aagagcccag atcacccatt ccgggttcac tccccgcctc cccaagtcag cagtcctagc     780

ccc                                                                   783

## Description

### Field of the invention

The present invention relates to the field of breast cancer. In particular, it relates to the selection of a breast cancer treatment regimen.

### Background of the invention

### Cancer

Cancer is one of the most common causes of disease and death in the western world. In general, incidence rates increase with age for most forms of cancer. As human populations continue to live longer, due to an increase of the general health status, cancer will affect an increasing number of individuals. The cause of most common cancer types is still at large unknown, although there is an increasing body of knowledge providing a link between environmental factors (dietary, tobacco smoke, UV radiation etc) as well as genetic factors (germ line mutations in "cancer genes" such as p53, APC, BRCA1, XP etc) and the risk for development of cancer.

No definition of cancer is entirely satisfactory from a cell biological point of view, despite the fact that cancer is essentially a cellular disease and defined as a transformed cell population with net cell growth and anti-social behavior. Malignant transformation represents the transition to a malignant phenotype based on irreversible genetic alterations. Although this has not been formally proven, malignant transformation is believed to take place in one cell, from which a subsequently developed tumor originates (the "clonality of cancer" dogma). Carcinogenesis is the process by which cancer is generated and is generally accepted to include multiple events that ultimately lead to growth of a malignant tumor. This multi-step process includes several rate-limiting steps, such as addition of mutations and possibly also epigenetic events, leading to formation of cancer following stages of precancerous proliferation. The stepwise changes involve accumulation of errors (mutations) in vital regulatory pathways that determine cell division, asocial behavior and cell death. Each of these changes may provide a selective Darwinian growth advantage compared to surrounding cells, resulting in a net growth of the tumor cell population. It is important to emphasize that a malignant tumor does not only consist of the transformed tumor cells themselves but also surrounding normal cells which act as a supportive stroma. This recruited cancer stroma consists of connective tissue, blood vessels and various other normal cells, e.g. inflammatory cells, which act in concert to supply the transformed tumor cells with signals necessary for continued tumor growth.

The most common forms of cancer arise in somatic cells and are predominantly of epithelial origin, e.g. prostate, breast, colon, urothelial and skin, followed by cancers originating from the hematopoetic lineage, e.g. leukemia and lymphoma, neuroectoderm, e.g. malignant gliomas, and soft tissue tumors, e.g. sarcomas.

### Cancer diagnostics and prognostics

Microscopic evaluation of a tissue section taken from a tumor remains the golden standard for determining a diagnosis of cancer. For microscopic diagnosis, biopsy material from suspected tumors is collected and examined under the microscope. To obtain a firm diagnosis, the tumor tissue is fixated in formalin, histo-processed and paraffin embedded. From the resulting paraffin block, tissue sections can be produced and stained using both histochemical, i.e. hematoxylin-eosin staining, and immunohistochemical methods. The surgical specimen is then evaluated with pathology techniques, including gross and microscopic analysis. This analysis forms the basis for assigning a specific diagnosis, i.e. classifying the tumor type and grading the degree of malignancy, of a tumor.

Malignant tumors can be categorized into several stages according to classification schemes specific for each cancer type. The most common classification system for solid tumors is the tumor-node-metastasis (TNM) staging system. The T stage describes the local extent of the primary tumor, i.e. how far the tumor has invaded and imposed growth into surrounding normal tissues, whereas the N stage and M stage describe how the tumor has developed into metastasis, with the N stage describing spread of tumor to lymph nodes and the M stage describing growth of tumor in other distant organs. Early stages include: T0-1, N0, M0, representing localized tumors with negative lymph nodes. More advanced stages include: T1-4, N0-4, M0, localized tumors with more widespread growth and T1-4, N1-4, M0, tumors that have metastasized to lymph nodes and T1-4, N1-4, M1, tumors with a metastasis detected in a distant organ. Staging of tumors is based on several forms of examinations, including surgical, radiological and histopathological analyses. In addition to the staging, there is also a classification system to grade the level of malignancy for most tumor types. The grading systems rely on morphological assessment of a tumor tissue sample and are based on the microscopic features found in a given tumor. These grading systems may be based on the degree of differentiation, proliferation and atypical appearance of the tumor cells. Examples of generally employed grading systems include Gleason grading for prostatic carcinomas and Elston-Ellis grading for breast carcinomas.

Accurate staging and grading is crucial for a correct diagnosis and provides an instrument to predict prognosis. The diagnostic and prognostic information for a specific tumor subsequently determines an adequate therapeutic strategy for the given cancer patient. The most commonly used method, in addition to histochemical staining of tissue sections, to obtain more information regarding a tumor is immunohistochemistry. Immunohistochemistry allows for the detection of protein expression patterns in tissues and cells using specific antibodies. The use of immunohistochemistry in clinical diagnostics allows for the detection of immunoreactivity in different cell populations, in addition to the information regarding tissue architecture and cellular morphology that is assessed from the histochemically stained tumor tissue section. Immunohistochemistry can be important to support the accurate diagnosis, including staging and grading, of a primary tumor as well as in the diagnostics of metastases of unknown origin. The most commonly used antibodies in clinical practice today include antibodies against cell type "specific" proteins, e.g. PSA (prostate), MelanA (melanocytes), Thyroglobulin (thyroid gland) and antibodies recognizing intermediate filaments (epithelial, mesenchymal, glial) cluster of differentiation (CD) antigens (hematopoetic, sub-classification of lympoid cells) and markers of malignant potential, e.g. Ki67 (proliferation), p53 (commonly mutated tumor suppressor gene), HER-2 (growth factor receptor).

Aside from immunohistochemistry, the use of *in situ* hybridization for detecting gene amplification and gene sequencing for mutation analysis are evolving technologies within cancer diagnostics. In addition, global analysis of transcripts, proteins or metabolites all add important information. However, most of these analyses still represent basic research and have yet to be evaluated and standardized for the use in clinical medicine.

In order for physicians to give a cancer patient the right type of treatment as early as possible, the provision of new molecular markers that allows for more accurate stratification of cancer patients into different risk categories is crucial. We are still far a way from this type of individually tailored treatment. In summary, there is a great demand for new means to advance prognostics and staging of cancer.

### Breast cancer

Breast cancer is the second most common form of cancer worldwide and by far the most frequent cancer of women. Data from the GLOBOCAM 2002 database presented by Parkin *et al* reveal 1.15 million new cases in 2002 and 0.41 million deaths during the same period (Parkin et al (2007) CA Cancer J Clin 55, 74-108). If detected at an early stage, the prognosis is relatively good for a patient living in a developed country, with a general five-year survival rate of 73%, compared to 57% in a developing country. The incidence is slowly increasing and about one in every nine women in the developed world will get breast cancer in her lifetime. Although lifestyle changes related to female steroid hormones, including exposure to exogenous hormones, affect the risk of developing breast cancer, these factors only make up for a small fraction of the etiology, and the gain of preventive manipulation would probably be low. The decreased mortality is due to earlier detection by mammography screening and the use of modern adjuvant systemic treatment.

### Treatment

Since its introduction in the late seventies, breast-conserving therapy, combining breast conserving surgery and postoperative radiotherapy, has become the primary treatment of choice in women where radical removal of the tumor can be combined with a good cosmetic result. Mastectomy is still preferable in some patients, i.e. women with small breasts, large tumors (> 4 cm) or multifocal/multicentric disease.

Axillary dissection is primarily performed for diagnostic purposes and removal of at least 10 lymph nodes gives a good staging guidance with 97-98% sensitivity (Axelsson (1992) Eur J Cancer 28A:1415-8; Recht and Houlihan (1995) Cancer 6(9):1491-1512) However, the next step towards minimal surgery in the treatment of primary cancer has been the introduction of the sentinel node biopsy technique with mapping of axillary lymph nodes instead of axillary lymph node clearance, which is associated with a high complication rate. This technique was introduced as a consequence of the knowledge that most of the lymphatic drainage to the axilla from the breast initially passes through one (or a few) lymph node(s)- the sentinel node(s)-supporting that analysis of this lymph node may be a sufficient indicator of axillary node status (Veronesi (2003) New Engl J Med 349(6): 546-53.)

The concept of breast cancer as a systemic disease, i.e. the presence of disseminating micro-metastases at the time of diagnosis that may explain treatment failure after locoregional therapy, paved the way for adjuvant randomized trials in the 1970s, including endocrine therapy and chemotherapy. Adjuvant polychemotherapy is standard treatment for hormone-receptor negative patients with high risk of recurrence, irrespective of nodal status. A beneficial effect on both overall- and relapse-free survival has been demonstrated, especially in premenopausal patients (EBCTCG (1998) Lancet 352(9132; 930-42). For patients with hormone-responsive disease, i.e. estrogen receptor (ER) and/or progesterone receptor (PR) positive disease, adjuvant polychemotherapy has been delivered in combination with endocrine therapy as sequential chemo-endocrine therapy. Adjuvant chemotherapy also induces amenorrhea, causing a secondary endocrine effect in addition to the cytotoxic (Pagani (1998) Eur J Cancer 34(5):632-40).

Endocrine therapy is recommended for patients with hormone receptor positive tumors irrespective of age, stage and menopausal status.

In hormone-responsive premenopausal patients, ovarian ablation by surgery or irradiation, or ovarian suppression by LHRH agonists are efficient adjuvant treatment modalities (Emens (2003) Clin Ca Res (1 Pt 2): 468S-94S). In postmenopausal patients, ovarian ablation has no place, since the primary source of estrogen is not from ovarian synthesis but from the conversion of androstenedione to estrone and estradiol in peripheral tissues including the breast.

Tamoxifen is a selective estrogen receptor modulator (SERM) with an agonistic effect on the ER, making it a suitable treatment for advanced breast cancer in both pre- and postmenopausal women. Five years of tamoxifen as adjuvant treatment after primary surgery clearly reduces the breast cancer mortality in patients with ER positive (ER+) tumors, irrespective of lymph node status (EBCTCG (1998) Lancet 351(9114):1451-67). While tamoxifen has a protective effect against cardiovascular disease, the risk of developing endometrial cancer is increased, due to an agonistic effect on the ER in the endometrium (EBCTCG (2005) Lancet 365(9472):1687-717)

Aromatase inhibitors (Als) function by inhibiting aromatase, the enzyme converting androgens into estrogens. Als are not suitable for treatment of premenopausal women, as it stimulates the ovaries to an increased androgen production through the hypothalamus and pituitary gland. Als can be given as adjuvant treatment to postmenopausal women, either alone or following tamoxifen treatment and they have been shown to significantly reduce the mortality, possibly even more if given alone (Howell (1995) Lancet 345(8941):29-30, Ellis and Rigden (2006) Curr Med Res Opin 22(12):2479-87, Coates (2007) J Clin Oncol 25(5):486-92). However, this therapy is relatively new and the long-term side effects are not yet fully known (Buzdar (2006) Lancet Oncol 7(8):633-43), but the most important are cardiovascular complications and osteoporosis.

Newly developed pure anti-estrogens such as fulvestrant, which completely blocks the ER, are currently only used in advanced breast cancer and not in the adjuvant setting (Rutqvist (2004) Best Pract Res Clin Endocrinol Metab 18(1): 81-95).

Adjuvant endocrine therapy has no place in hormone receptor negative breast cancer, although some studies indicate that some ER negative (ER-), i.e. ERα negative (ERα-), tumors respond to tamoxifen treatment (EBCTCG (1998) Lancet 351:1451-1467)

The HER2/neu gene is over expressed in about 20% of all and in up to 70% of low differentiated breast cancers (Berger (1988) Cancer Res 48(5): 1238-43, Borg (1990) Cancer Res 50(14): 4332-7). Patients with HER2 overexpressing tumors may benefit from treatment with the monoclonal antibody trastuzumab. Experimental data support a relationship between HER2 overexpression and resistance to endocrine treatment (Shou (2004) J Natl Cancer Inst 96(12):926-35) while clinical data are not consistent (Borg (1994) Cancer Lett 81(2):137-44, De Placido (2003) Clin Ca Res 9(3):1039-46, Rydén et al. (2005) J Clin Oncol 23(21):4695-704).

### Breast cancer diagnostics

Morphologic criteria are still essential for the establishment of a breast cancer diagnosis, both *in situ* and invasive cancer, and although such criteria are more or less subjective, they are not easily substituted. Among invasive breast carcinomas, *invasive ductal carcinoma* is the most common tumor type (∼80 %) and *lobular carcinoma* is the second largest entity (∼10-15%). *Tubular* and *medullary* carcinomas are other distinct types with lower prevalence (WHO, Histological typing of breast tumors, in International histological classification of tumors no:2, 1981, WHO, Geneva).

### Prognostic and treatment predictive factors

A correct histological classification of the tumor type may be of prognostic relevance, since certain subtypes, such as medullary carcinomas, in general have a more favorable prognosis. Nevertheless, assessment of the histological grade using the Nottingham Histological Grade (NHG) system is still an important prognostic tool (Elston and Ellis, Pathological prognostic factors in breast cancer: experience from a large study with long-term follow-up. Histopathology, 1991, 19(5):403-10, Sundquist (1999) Breast Cancer Res Treat 53(1):1-8).

The majority of breast cancers are hormone receptor responsive, i.e. express the ER and/or PR. The action of estrogen is mediated by the two receptors, ERα and ERβ. ERα together with PR are routinely assessed in order to select patients for endocrine therapy, and tumors with >10 % nuclear positivity are considered positive. ERα is today considered the most important predictor of tamoxifen response. However, there are studies that indicate that PR positivity may even be a more powerful predictor of tamoxifen response than ERα. A study of premenopausal patients randomly treated with tamoxifen revealed that a high, >75% nuclear fraction, PR level was significantly correlated with an increased recurrence free and overall survival, irrespectively of ERα status. At a lower PR level, <75%, no positive effect was observed (Stendahl el al (2006) Clin Cancer Res 12:4614-18). Yu et al recently studied the predictive value of PR for adjuvant endocrine therapy, and found that older patients (>=60 years) with ERα+/PR+ tumors had a significantly longer disease free survival when treated with tamoxifen than patients that received no adjuvant treatment. In younger patients (<60 years), no significant effect was observed (Yu el al (2007) The Breast 16:307-315). Interestingly, a report from the ATAC trial (postmenopausal women treated with arimidex, tamoxifen or in combination), showed that the recurrence rate was halved for anastrozole-treated patients with ERα+/PR- tumors over the follow-up period of 6 years, compared to patients treated with tamoxifen (Dowsett et al (2005) J Clin Oncol 23(30:7512-7).

The role of ERβ in breast cancer is not yet fully clarified, although recent studies implicate that ERβ-expression may be associated with a better tamoxifen response (Borgquist et al, in press JCP), particularly in ERα-tumors (Gruvberger-Saal et al (2007) Clin Cancer Res. 13:1987-1994). Determination of ERβ is today not considered clinically relevant.

A major problem to day is that 30-40% of the ERα positive (ERα+) patients do not respond to tamoxifen treatment (Riggins et al (2007) Cancer Letters 1:1-24, Gruvberger-Saal et al (2007) Clin Cancer Res. 13:1987-1994), which results in unnecessary treatment. In addition, a fraction of the ERα-patients do respond to tamoxifen treatment, and the reason for that is currently not known. Gruvberger-Saal suggests that ERβ-expression may be a positive predictor of tamoxifen response in ERα- patients (Gruvberger-Saal et al (2007) Clin Cancer Res. 13:1987-1994.

HER2 status is also assessed routinely, primarily by IHC and in cases with moderate expression, gene amplification status is determined by FISH analysis. Patients with a HER2 positive tumor may be treated with Trastuzumab (Herceptin).

Breast cancer is a truly heterogeneous disease and despite the increasing understanding of its nature, the arsenal of available prognostic and treatment predictive markers is still not sufficient and some patients will therefore receive unnecessary treatment while others will not get sufficient or even wrong treatment. Additional molecular markers are needed in order to better define different subgroups of breast cancer and increase the options for tailored therapies.

### CRABP2

The gene encoding Cellular retinoic acid binding protein 2 (CRABP2) was initially isolated from human skin (Åström et al (1991) J Biol Chem 266:17662-6). Expression analysis revealed a specific expression in skin, which increased after application of retinoic acid (RA). In addition, studies in rats revealed expression in uterus and ovary (Wardlaw et al (1997) Biol Reprod 56:125-132). It has been demonstrated that the CRABP2 protein is present in both cytoplasm and the nuclei of cells. Later on, it was demonstrated that CRABP2 is responsible for channeling retinoic acid (RA) from the cytoplasm through the membrane and to the RA receptor (RAR) located n the nucleus. RA is well known to promote cell proliferation, differentiation and apoptosis through the RA receptor. Retinoids are also capable of suppressing tumor promotion in several types of cancer including breast carcinomas (Lotan (1996) FASEB 10:1031-9). Over expression of CRABP2 has been shown to suppress tumor development whereas loss of expression results in resistance to RA dependent growth inhibition (Manor et al. (2003) Cancer Res. 63:4426-4433).

A clinical value for CRABP2 in cancer diagnostics has been reported at several occasions such as in patent W02004111650, where CRABP2 is presented as a marker for early detection and diagnosis of breast cancer. The method includes collection of a liquid sample from a patient suspected to suffer from breast cancer. High levels of CRABP2 indicate presence of a tumor. CRABP2 analysis is also suggested to be part of a following up program after surgery, where an increasing CRABP2 level after 3 months indicates tumor recurrence. They also present a possibility of using CRABP2 in combination with other markers e.g. cancer antigen (CA) 15-3, to increase the sensitivity of the diagnosis. A method for diagnosing early breast cancer is presented in patent WO02077176. Here a proteomic approach to identify hundreds of proteins differentially regulated in normal and cancerous tissue is presented. CRABP2 was found to be upregulated in ductal carcinoma in situ DCIS and breast tumor tissue, compared to normal breast tissue. The authors suggest treating the cancer with a pharmaceutical compound meanwhile monitoring the protein level, and further, considering the cancer as treated when a normal level is reached.

### Disclosure of the invention

Importantly, the studies referred to above provide no suggestions concerning the use of the CRABP2 protein as a predictive tool for breast cancer treatment and/or therapy. Neither do any of those studies suggest the use of CRABP2 as an endocrine treatment indicator for breast cancer.

It is an object of the present invention to provide improvements relating to the selection of an appropriate breast cancer treatment and predicting the outcome of the same.

For this and other objects apparent to the skilled person from the present disclosure, the present invention provides, in its different aspects, new means for determining, or supporting a decision regarding, whether a mammalian subject having a breast cancer should undergo endocrine treatment, and for treatment of a breast cancer. The present invention is defined by the appending claims.

Thus, according to a first aspect, the present invention provides a method for determining whether a mammalian subject having a breast cancer should undergo an endocrine treatment, comprising the steps of:
a) providing a sample earlier obtained from said subject;
b) evaluating the amount of CRABP2 protein present in at least part of said sample, and determining a sample value corresponding to said amount;
c) comparing the sample value obtained in step b) with a reference value; and, if said sample value is higher than said reference value,
d) concluding that the subject should undergo endocrine treatment.

Regarding step b), an increase in the amount of CRABP2 protein typically results in an increase in the sample value, and not the other way around.

This first aspect of the present invention is based on the previously unrecognized fact that the expression of CRABP2 protein in samples earlier obtained from a subject having a breast cancer may serve as an indicator of response to an endocrine treatment of the subject. More particularly, the present invention identifies for the first time, in patients suffering from breast cancer, a correlation between a value of CRABP2 protein on the one hand and the survival after an endocrine treatment on the other. Typically, high CRABP2 values have been shown to correlate with a responsiveness response to endocrine treatment. The present invention, based on CRABP2 protein expression as an breast cancer treatment indicator, has a number of benefits. Firstly, it provides an additional, or alternative, tool for predicting whether a patient is likely to respond to endocrine treatment. Secondly, it identifies a subgroup of hormone receptor negative patients, that, contrary to earlier believes, seem to respond to endocrine treatment. Consequently, the invention may provide for accurate treatment of a previously undertreated group. Thirdly, the invention identifies another subgroup of hormone receptor negative patients, where an endocrine treatment seem to have a negative effect on survival.

In an alternative embodiment of the first aspect, there is provided a method for determining whether a mammalian subject having a breast cancer is likely to benefit from an endocrine treatment, comprising the steps of:
a) providing a sample earlier obtained from said subject;
b) evaluating the amount of CRABP2 protein present in at least part of said sample, and determining a sample value corresponding to said amount;
c) comparing the sample value obtained in step b) with a reference value; and, if said sample value is higher than said reference value,
d) concluding that the subject is likely to benefit from an endocrine treatment.

Regarding step b), an increase in the amount of CRABP2 protein typically results in an increase in the sample value, and not the other way around.

In the context of the present disclosure, "likely to benefit from an endocrine treatment" refers to a having a higher probability of survival or recovery if undergoing an endocrine treatment than if not undergoing an endocrine treatment. In this context, "recovery" refers to return from a breast cancer sate to a breast cancer free state. The "survival" may be a recurrence free survival or a breast cancer specific survival. Further, the "recovery" may be a recurrence free recovery. Also, the "higher probability" may be a probability benefit at five years, ten years or 15 years of at least 5 %, such as at least 10 %.

In another alternative embodiment of the first aspect, there is provided a method for determining whether a sample value corresponding to an amount of CRABP2 protein present in at least part of a sample earlier obtained from a mammalian subject having a breast cancer is in favour of a decision to give an endocrine treatment to said subject, comprising the steps of:
a) providing said sample earlier obtained from said subject;
b) evaluating said amount of CRABP2 protein present in at least part of said sample, and determining said sample value corresponding to said amount;
c) comparing the sample value obtained in step b) with a reference value; and, if said sample value is higher than said reference value
d) concluding that said sample value is in favor of a decision to treat said subject with an endocrine treatment.

Regarding step b), an increase in the amount of CRABP2 protein typically results in an increase in the sample value, and not the other way around.

When deciding on a suitable treatment regimen for a breast cancer patient, the physician responsible for the treatment may take several parameters into account, such as the result of an immunohistochemical evaluation, patient age, menopausal status, general condition and medical history, such as breast cancer history. To be guided in the decision, the physician may perform CRABP2 test, or order a CRABP2 test performed, according to the above embodiment to find out whether a sample value is in favor of a decision to give an endocrine treatment to the patient.

In another alternative embodiment of the first aspect, there is provided a method for determining whether a prognosis for an endocrine treatment of a mammalian subject having or suspected of having a breast cancer is better than a reference prognosis, comprising the steps of:
a) providing a sample earlier obtained from said subject;
b) evaluating the amount of CRABP2 protein present in at least part of said sample, and determining a sample value corresponding to said amount;
c) comparing the sample value obtained in step b) with a reference value; and, if said sample value is higher than said reference value,
d) concluding that the prognosis for endocrine treatment of said subject is better than said reference prognosis.

Regarding step b), an increase in the amount of CRABP2 protein typically results in an increase in the sample value, and not the other way around.

For example, the reference prognosis may be a likelihood of survival, such as five-year survival, ten-year survival or 15-year survival. As an example, the reference prognosis may be specific to the hormone receptor status, such as the ER status and/or the PR status, of the subject, i.e. one reference prognosis may apply to hormone receptor positive, e.g. ER+ and/or PR+, subjects only and another reference prognosis may apply to hormone receptor negative, e.g. ER- or PR-, subjects only. The man skilled in the art understands how to determine a hormone receptor status of the subject. Also, examples of hormone receptor status determinations are given below in connection with the endocrine treatment product aspects.

A physician responsible for the treatment of a subject suffering from breast cancer may, with the knowledge of a prognosis of an endocrine treatment of the subject, decide on whether to give the subject the endocrine treatment or not.

In the present disclosure, different CRABP2 values (sample values) corresponding to various prognoses for an endocrine treatment are presented. In the above method, the sample value is compared to a reference value associated with a reference prognosis, and if the sample value is higher than the reference value, it is concluded that the prognosis for an endocrine treatment of the subject is better than the reference prognosis.

Consequently, the method according to the above embodiment may be adapted to a given reference value. In such case, starting from a given sample value which under certain circumstances is considered to be relevant, a reference value which is lower than that sample value, may be selected. Subsequently, a reference prognosis being associated with that reference value may be established. Guided by the present disclosure, the person skilled in the art understands how to establish a reference prognosis which corresponds to a given reference value. For example, the relation between sample values and survival data in a group of breast cancer patients may be examined as in Examples, section 4, below, and the procedure described therein may be adapted to the given reference value. Then, a prognosis corresponding to the given reference value may be selected as the reference prognosis.

In embodiments of the above method aspect, the reference prognosis may be a likelihood of five-year recurrence free survival of 57 % or higher, such as 60 % or higher, such as 65 % or higher, such as 70 % or higher, such as 75 % or higher, such as 80 % or higher, such as 85 % or higher, such as 90 % or higher, such as 95 % or higher.

In other embodiments of the above method aspect, the reference prognosis may be a likelihood of ten-year recurrence free survival of 47 % or higher, such as 50 % or higher, such as 55 % or higher, such as 60 % or higher, such as 65 % or higher, such as 70 % or higher, such as 75 % or higher, such as 80 % or higher, such as 85 % or higher, such as 90 % or higher, such as 95 % or higher.

Further, in embodiments of the above method aspect, wherein the breast cancer is an ER- breast cancer, the reference prognosis may be a likelihood of five-year recurrence free survival of 50 % or higher, such as 55 % or higher, such as 60 % or higher, such as 65 % or higher, such as 70 % or higher, such as 75 % or higher, such as 80 % or higher, such as 85 % or higher, such as 90 % or higher, such as 95 % or higher.

In other embodiments of the above method aspect, wherein the breast cancer is an ER- breast cancer, the reference prognosis may be a likelihood of ten-year recurrence free survival of 45 % or higher, such as 50 % or higher, such as 55 % or higher, such as 60 % or higher, such as 65 % or higher, such as 70 % or higher, such as 75 % or higher, such as 80 % or higher, such as 85 % or higher, such as 90 % or higher, such as 95 % or higher.

Further, in embodiments of the above method, wherein the breast cancer is an ER- breast cancer, the reference prognosis may be a likelihood of five-year breast cancer specific survival of 60 % or higher, such as 65 % or higher, such as 70 % or higher, such as 75 % or higher, such as 80 % or higher, such as 85 % or higher, such as 90 % or higher, such as 95 % or higher.

In other embodiments of the above method, wherein the breast cancer is an ER- breast cancer, the reference prognosis may be a likelihood of ten-year breast cancer specific survival of 50 % or higher, such as 55 % or higher, such as 60 % or higher, such as 65 % or higher, such as 70 % or higher, such as 75 % or higher, such as 80 % or higher, such as 85 % or higher, such as 90 % or higher, such as 95 % or higher.

Further, starting from relevant CRABP2 reference values, the reference prognosis may be based on a previous prognosis obtained by an examination of the same patient or population of patients. Alternatively, or as a complement, the reference prognosis may be based on a background risk in the general population, a statistical prognosis/risk or an assumption based on an examination of the patient. Such examination may comprise the patient's age, general condition, sex, race and/or medical status and history, such as cancer history or cancer status. For example, a reference prognosis may be established by a physician taking into regard the patient's cancer history, the stage of the tumor, the morphology of the tumor, the location of the tumor, the presence and spread of metastases and/or further cancer characteristics.

The identified correlation between high CRABP2 protein expression and a responsiveness to an endocrine treatment may also form the basis for a decision to apply a specific regimen for treatment of the subject. Thus, in a second aspect, the present invention provides a method for treatment a breast cancer in a subject in need thereof, comprising the steps of:
a) providing a sample from said subject;
b) evaluating the amount of CRABP2 protein present in at least part of said sample, and determining a sample value corresponding to said amount;
c) comparing the sample value obtained in step b) with a reference value; and, if said sample value is higher than said reference value,
d) treating said subject with an endocrine treatment regimen.

Regarding step b), an increase in the amount of CRABP2 protein typically results in an increase in the sample value, and not the other way around.

In embodiments of the methods of the above aspects, the breast cancer may be a hormone receptor negative breast cancer, such as an ER- or PR- breast cancer, an ER- breast cancer, a PR- breast cancer, or an ER- and PR- breast cancer. For example, the breast cancer may be previously diagnosed as hormone receptor negative. Hormone receptor status, e.g. ER status or PR status, may be assessed according to any method known to the skilled artisan. For example, the assessment of hormone receptor status may be performed as described below in connection with the endocrine treatment product aspects.

Traditionally, subjects with hormone receptor negative breast cancers, especially ER- breast cancers, have not been treated with endocrine treatments. However, as shown in the present disclosure, the survival rates in the subgroup of subjects with hormone receptor negative, e.g. ER- or PR-, ER-, PR-, or ER- and PR-, breast cancers having high CRABP2 values were increased with an endocrine treatment. Consequently, the inventors have identified new groups of subjects that may be treated with an endocrine treatment.

Further, the survival rates in the subgroups of subjects having hormone receptor negative breast cancers having with low CRABP2 values were not increased with an endocrine treatment. Surprisingly, the survival rates of the CRABP2 low patients having hormone receptor negative breast cancers that were treated with an endocrine treatment were actually lower than those of a corresponding, non-treated control group.

Accordingly, there is also provided a method for treatment of a hormone receptor negative breast cancer in a subject in need thereof, comprising
a) providing a sample from said subject;
b) evaluating the amount of CRABP2 protein present in at least part of said sample, and determining a sample value corresponding to said amount;
c) comparing the sample value obtained in step b) with a reference value; and, if said sample value is lower than or equal to said reference value,
d) treating said subject with a non-endocrine treatment regimen.

For example, the hormone receptor negative breast cancer may be ER- or PR-, ER-, PR-, or ER- and PR-.

For example, the non-endocrine treatment may be chemotherapy, radiation therapy or a combination thereof. Also, the non-endocrine treatment may for example be an antibody treatment, such as an anti-HER2 antibody treatment if said subject is HER2 positive.

In the context of the present disclosure, an "endocrine treatment" refers to a systemic treatment with an anti-estrogenic effect. Further, "anti-estrogenic effect" refers to suppression of estrogen production or inhibition of estrogen effects in the body. In the art, an endocrine treatment is sometimes referred to as an anti-hormonal treatment.

Step b) of the methods of the above aspects involve evaluating the amount of CRABP2 protein present in at least part of the sample, and determining a sample value corresponding to the amount. The "at least part of the sample" refers to a relevant part, or relevant parts, of the sample. The person skilled in the art understands which part or parts that are relevant under the circumstances present when performing the method. For example, if evaluating at a sample comprising cells, the skilled person may only consider the tumor cells, or only the nuclei of tumor cells, of the sample.

Further, in step b) an amount is evaluated and a sample value corresponding to the amount is determined. Consequently, an exact measurement of the amount of CRABP2 protein is not required for obtaining the sample value. For example, the amount of CRABP2 protein may be evaluated by visual inspection of a stained sample and the sample value may then be categorized as a high or low based on the evaluated amount.

In embodiments of the methods of the above aspects, the endocrine treatment may be selected from the group consisting of a selective estrogen receptor modulator (SERM) treatment, an aromatase inhibitor treatment, and a steroidal estrogen receptor antagonist treatment. The SERM treatment may be a treatment with e.g. toremifene, raloxifene, droloxifene, arzoxifene or tamoxifene. The aromatase inhibitor treatment may be a treatment with e.g. anastrozole, letrozole and exemestane. Further, the steroidal estrogen receptor antagonist treatment may be a treatment with fulvestrant.

A SERM has either an agonistic or antagonistic effect depending on the target tissue. For example, a SERM may act as an antagonist in breast and as an agonist in uterus.

In embodiments of the methods of the above aspects, the endocrine treatment may be a SERM treatment, such as a SERM treatment selected from treatments with toremifene, raloxifene, droloxifene, arzoxifene or tamoxifene. For example, in embodiments of the methods of the above aspects, the endocrine treatment may be tamoxifen treatment.

In embodiments of the methods of the above aspects, the sample may be a body fluid sample, such as blood, plasma, serum, cerebral fluid, urine, and exudate. Alternatively, the sample may be a cytology sample or a stool sample.

In further embodiments of the methods of the above aspects, the sample may be a tissue sample. As an example, the tissue sample may bederived from a primary breast tumor or secondary tumour (metastasis) of the subject. Further, the tissue sample may be a tumor sample from a breast of the subject.

In embodiments of the methods of the above aspects, the evaluation of step b) may be limited to evaluating the amount of CRABP2 protein expression in tumor cells, of said tissue sample. The inventors have found that CRABP2 is expressed in the nucleus and in the cytoplasm. Consequently, in embodiments of the methods of the above aspects, the evaluation of step b) may be limited to evaluating the amount of CRABP2 protein expression in the nucleus or cytoplasm of tumor cells of said tissue sample. For example, the evaluation of step b) may be limited to evaluating the amount of nuclear CRABP2 protein expression in tumor cells of said tissue sample.

In embodiments of the methods of the above aspects, the subject may be a human female.

Further, in embodiments of the methods of the above aspects, the subject may be a premenopausal female.

Also, in embodiments of the methods of the above aspects, the breast cancer may be a TNM stage II (i.e. pT2 N0 M0, pT1 N1 M0 or pT2 N1 M0) breast carcinoma.

A reference value found to be relevant for indicating a response to anendocrine treatment, for use as comparison with the sample value from the subject, may be provided in various ways. In embodiments of the methods of the above aspects, the reference value may correspond to the amount of CRABP2 protein expression in healthy tissue of the subject of the method. As another example, the reference value may be provided by the amount of CRABP2 protein expression measured in a standard sample of normal tissue from another, comparable subject. As another example, the reference value may be provided by the amount of CRABP2 protein expression measured in a reference sample comprising tumor cells, such as a reference sample of tumor tissue. As another example, the reference value may be provided by the amount of CRABP2 protein measured in a reference sample comprising cells expressing a predetermined amount of CRABP2 protein.

As another example the reference value may be provided by the amount of CRABP2 protein expression measured in a reference sample comprising cell lines, such as cancer cell lines, expressing a predetermined, or controlled, amount of CRABP2 protein. The person skilled in the art understands how to provide such cell lines, for example guided by the disclosure of Rhodes et al. (2006) The biomedical scientist, p 515-520.

Accordingly, in embodiments of the methods of the above aspects, the reference value may be a predetermined value corresponding to the amount of CRABP2 protein in a reference sample.

In the context of the present disclosure, the terms "sample value" and "reference value" are to be interpreted broadly. The quantification of CRABP2 protein to obtain these values may be done via automatic means, via a scoring system based on visual or microscopic inspection of samples, or via combinations thereof. However, it is also possible for a skilled person, such as a person skilled in the art of histopathology, to determine the sample and reference values merely by inspection of e.g. tissue slides that have been stained for CRABP2 protein expression. The determination of the sample value being higher than the reference value may thus correspond to the determination, upon visual or microscopic inspection, that a sample tissue slide is more densely stained and/or exhibit a larger fraction of stained cells than is the case for a reference tissue slide. In this case, the sample and reference values are thought of as mental values that the skilled person determines upon inspection and comparison.

The skilled person may categorize a sample as being high or low in CRABP2 protein expression, wherein the sample is categorized as high in CRABP2 protein expression if it contains more CRABP2 protein than a previously inspected reference sample. Such evaluation may be assisted by staining the sample, and, if necessary, the reference sample, with a staining solution comprising e.g. antibodies specific for CRABP2 protein.

One alternative for the quantification of CRABP2 expression in a sample is the determination of the fraction of cells in the sample that exhibit CRABP2 protein expression over a certain level. The fraction may for example be: a "cellular fraction", wherein the CRABP2 protein expression of the whole cells is taken into account; a "cytoplasmic fraction", wherein the CRABP2 protein expression of only the cytoplasms of the cells is taken into account, or the "nuclear fraction", wherein the CRABP2 protein expression of only the nuclei of the cells is taken into account. This determination may for example be performed as described below in the Examples, section 4, with reference to "nuclear fraction". The "nuclear fraction" corresponds to the percentage of relevant cells in a sample that exhibits a positive staining in the nucleus, wherein a medium or distinct and strong immunoreactivity in the nucleus is considered positive and no or faint immunoreactivity is considered negative. The person skilled in the art understands which cells that are relevant under the conditions present when performing the method. The relevant cells may for example be tumor cells. Further, the skilled artisan understands how to perform corresponding measurements employing the "cellular fraction" or the "cytoplasmic fraction". In embodiments of the methods of the above aspects, the criterion for the conclusion in step d) is a sample value for the nuclear fraction of CRABP2 positive cells, i.e. a "nuclear fraction", which is higher than the reference value of 1 %, such as higher than 5 %, such as higher than 10 %, such as higher than 15 %, such as higher than 20 %, such as higher than 25 %, such as higher than 30 %, such as higher than 35 %, such as higher than 40 %, such as higher than 50 %, such as higher than 55 %, such as higher than 60 %.

In alternative or complementing embodiments of the methods of the above aspects, the reference value of step c) is a nuclear fraction of 1 % or higher, such as 2 % or higher, such as 5 % or higher, such as 10 % or higher, such as 15 % or higher, such as 20 % or higher, such as 25 % or higher, such as 30 % or higher, such as 35 % or higher, such as 40 % or higher, such as 45 % or higher, such as 50 % or higher, such as 55 % or higher, such as 60 % or higher.

Another alternative for the quantification of CRABP2 expression in a sample is the determination of the over-all staining intensity of the sample. The intensity may for example be: a "cellular intensity", wherein the CRABP2 protein expression of the whole cells is taken into account; a "cytoplasmic intensity", wherein the CRABP2 protein expression of only the cytoplasms of the cells is taken into account, or the "nuclear intensity", wherein the CRABP2 protein expression of only the nuclei of the cells is taken into account. This determination may for example be performed as described below in the Examples, section 4, definition of "nuclear intensity". Consequently, nuclear intensity may be subjectively evaluated in accordance to standards used in clinical histo-pathological diagnostics. Outcome of a nuclear intensity determination is classified as: absent = no over-all immunoreactivity in relevant cells of the sample, weak = faint over-all immunoreactivity in relevant cells of the sample, moderate = medium over-all immunoreactivity in relevant cells of the sample, or strong = distinct and strong over-all immunoreactivity in relevant cells of the sample. The person skilled in the art understands which cells that are relevant under the conditions present when performing the method. The relevant cells may for example be tumor cells. Further, the skilled artisan understands how to perform corresponding measurements employing the "cellular intensity" or the "cytoplasmic intensity". In embodiments of the methods of the above aspects, the criterion for the conclusion in step d) may be a sample value for staining intensity of a sample, i.e. a nuclear intensity, which is higher than absent nuclear intensity, such as higher than weak intensity, such higher than moderate intensity. In alternative or complementing embodiments of the methods of the above aspects, the reference value of step c) may be an absent nuclear intensity of CRABP2 protein expression or higher, such as a weak nuclear intensity of CRABP2 protein expression or higher, such as moderate nuclear intensity of CRABP2 protein expression.

Further, in embodiments of the methods of the above aspects, the reference value may be constituted of two values, wherein the criterion for the conclusion in step d) is a sample value being higher than any of these two values. As an example, the reference value may be a nuclear fraction of 2 % or higher, or a weak nuclear intensity or higher. As another example, the reference value may be a nuclear fraction of 25 % or higher, or an absent nuclear intensity or higher. As another example, the reference value may be a nuclear fraction of 25 % or higher, or a weak nuclear intensity or higher. As another example, the reference value may be a nuclear fraction of 50 % or higher or a weak nuclear intensity or higher. As another example, the reference value may be a nuclear fraction of 25 % or higher or a moderate nuclear intensity or higher. As another example, the reference value may be a nuclear fraction of 50 % or higher or a moderate nuclear intensity or higher. The person skilled in the art realizes that other reference values being an intensity values or a fraction value also fall within the scope of the present invention.

Alternatively, in embodiments of the methods of the above aspects, the reference value may be a coincidence of a fraction value and an intensity value, such as a nuclear fraction value and a nuclear intensity value. As an example, the reference value may be a nuclear fraction of 2 % or higher, and a weak nuclear intensity or higher. As another example, the reference value may be a nuclear fraction of 25 % or higher, and an absent nuclear intensity or higher. As another example, the reference value may be a nuclear fraction of 25 % or higher and a weak nuclear intensity or higher. As another example, the reference value may be a nuclear fraction of 50 % or higher and a weak nuclear intensity or higher. As another example, the reference value may be a nuclear fraction of 25 % or higher and a moderate nuclear intensity or higher. As another example, the reference value may be a nuclear fraction of 50 % or higher and a moderate nuclear intensity or higher. The person skilled in the art realizes that other combinations of fractions and intensities also fall within the scope of the present invention. Consequently, the reference value may involve two, and possibly even more, criteria.

Guided by the present disclosure, and especially Examples, section 4 below, a person skilled in the art, e.g. a pathologist, understands how to perform the evaluation yielding a fraction, such as a cellular, cytoplasmic or nuclear fraction, or an intensity, such as a cellular, cytoplasmic or nuclear intensity. For example, the skilled artisan may use a reference sample comprising a predetermined amount of CRABP2 protein for establishing the appearance of a certain fraction or intensity.

However, a reference sample may not only be used for providing the actual reference value, but also for providing an example of a sample with an amount of CRABP2 that is higher than the amount corresponding to the reference value. As an example, in histological staining, such as in immunohistological staining, the skilled artisan may use a reference sample for establishing the appearance of a stained sample with an high amount of CRABP2 protein. Subsequently, the skilled artisan may assess the appearances of samples with lower amounts of CRABP2, such as the appearance of a sample with an amount of CRABP2 corresponding to the reference value. In other words, the skilled artisan may use a reference sample to create a mental image of a reference value corresponding to an amount of CRABP2 protein which is lower than that of the reference sample. Further, in these assessments, the skilled artisan may use another reference sample having a low amount of CRABP2, or essentially lacking CRABP2, for establishing the appearance of such sample, e.g. as a "negative reference".

Consequently, in the evaluation, the skilled artisan may use a reference sample for establishing the appearance of a sample with a high amount of CRABP2 protein. Such reference sample may be a sample comprising tissue expressing a high amount of CRABP2, such as tissue comprising surface epithelial cells from the vagina or vulva/anal.

Further, the reference sample may provide an example of a strong nuclear intensity (ni). With the knowledge of the appearance of a sample with strong nuclear intensity, the skilled artisan may then divide samples into the ni categories presented in Examples, section 4, below, i.e. absent, weak, moderate and strong. This division may be further assisted by a reference sample essentially lacking CRABP2 protein (negative reference), i.e. a reference sample providing an absent nuclear intensity. Also, the reference sample may provide an example of a sample with a nuclear fraction (nf) of 75 % or higher. With the knowledge of the appearance of a sample with more than 75 % positive cells, the skilled artisan may then evaluate the nuclear fraction of other samples having e.g. a lower percentage of positive cells.

The skilled person will recognize that the usefulness of the present invention is not limited to the quantification of any particular variant of the CRABP2 protein present in the subject in question, as long as the protein is encoded by the relevant gene and presents the relevant pattern of expression. As a non-limiting example, the CRABP2 protein has an amino acid sequence which comprises a sequence selected from:
i) SEQ ID NO:1; and
ii) a sequence which is at least 85 % identical to SEQ ID NO:1.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

As another non-limiting example, the CRABP2 protein has an amino acid sequence which comprises a sequence selected from:
i) SEQ ID NO:2; and
ii) a sequence which is at least 85 % identical to SEQ ID NO:2.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2.

In embodiments of the methods of the aspects above, the CRABP2 protein may be detected and/or quantified through the application to a sample of a detectable and/or quantifiable affinity ligand, which is capable of specific or selective interaction with the CRABP2 protein. The application of the affinity ligand is performed under conditions that enable binding of the affinity ligand to any CRABP2 protein in the sample. It is regarded as within the capabilities of those of ordinary skill in the art to select or manufacture the proper affinity ligand and to select the proper format and conditions for detection and/or quantification, once the connection between CRABP2 protein and breast cancer is known through the teaching of the present disclosure. Nevertheless, examples of affinity ligands that may prove useful, as well as examples of formats and conditions for detection and/or quantification, are given below for the sake of illustration.

Thus, in some embodiments, an affinity ligand is used, which is selected from the group consisting of antibodies, fragments thereof and derivatives thereof, i.e. affinity ligands based on an immunoglobulin scaffold. Antibodies comprise monoclonal and polyclonal antibodies of any origin, including murine, human and other antibodies, as well as chimeric antibodies comprising sequences from different species, such as partly humanized mouse antibodies. Polyclonal antibodies are produced by immunization of animals with the antigen of choice, whereas monoclonal antibodies of defined specificity can be produced using the hybridoma technology developed by Köhler and Milstein (Köhler G and Milstein C (1976) Eur. J. Immunol. 6:511-519). Antibody fragments and derivatives comprise Fab fragments, consisting of the first constant domain of the heavy chain (CH1), the constant domain of the light chain (CL), the variable domain of the heavy chain (VH) and the variable domain of the light chain (VL) of an intact immunoglobulin protein; Fv fragments, consisting of the two variable antibody domains VH and VL (Skerra A and Plückthun A (1988) Science 240:1038-1041); single chain Fv fragments (scFv), consisting of the two VH and VL domains linked together by a flexible peptide linker (Bird RE and Walker BW (1991) Trends Biotechnol. 9:132-137); Bence Jones dimers (Stevens FJ et al (1991) Biochemistry 30:6803-6805); camelid heavy-chain dimers (Hamers-Casterman C et al (1993) Nature 363:446-448) and single variable domains (Cai X and Garen A (1996) Proc. Natl. Acad. Sci. U.S.A. 93:6280-6285; Masat L et al (1994) Proc. Natl. Acad. Sci. U.S.A. 91:893-896), and single domain scaffolds like e.g. the New Antigen Receptor (NAR) from the nurse shark (Dooley H et al (2003) Mol. Immunol. 40:25-33) and minibodies based on a variable heavy domain (Skerra A and Plückthun A (1988) Science 240:1038-1041).

Polyclonal and monoclonal antibodies, as well as their fragments and derivatives, represent the traditional choice of affinity ligands in applications requiring selective biomolecular recognition, such as in the detection and/or quantification of CRABP2 protein according to the method aspects above. However, those of skill in the art know that, due to the increasing demand of high throughput generation of specific binding ligands and low cost production systems, new biomolecular diversity technologies have been developed during the last decade. This has enabled a generation of novel types of affinity ligands of both immunoglobulin as well as non-immunoglobulin origin that have proven equally useful as binding ligands in biomolecular recognition applications and can be used instead of, or together with, immunoglobulins.

The biomolecular diversity needed for selection of affinity ligands may be generated by combinatorial engineering of one of a plurality of possible scaffold molecules, and specific and/or selective affinity ligands are then selected using a suitable selection platform. The scaffold molecule may be of immunoglobulin protein origin (Bradbury AR and Marks JD (2004) J. Immunol. Meths. 290:29-49), of non-immunoglobulin protein origin (Nygren PÅ and Skerra A (2004) J. Immunol. Meths. 290:3-28), or of an oligonucleotide origin (Gold L et a/ (1995) Annu. Rev. Biochem. 64:763-797).

A large number of non-immunoglobulin protein scaffolds have been used as supporting structures in development of novel binding proteins. Non-limiting examples of such structures, useful for generating affinity ligands against CRABP2 for use according to the present disclosure, are staphylococcal protein A and domains thereof and derivatives of these domains, such as protein Z (Nord K et al (1997) Nat. Biotechnol. 15:772-777); lipocalins (Beste G et al (1999) Proc. Natl. Acad. Sci. U.S.A. 96:1898-1903); ankyrin repeat domains (Binz HK et al (2003) J. Mol. Biol. 332:489-503); cellulose binding domains (CBD) (Smith GP et al (1998) J. Mol. Biol. 277:317-332; Lehtiö J et al (2000) Proteins 41:316-322); γ crystallines (Fiedler U and Rudolph R, WO01/04144); green fluorescent protein (GFP) (Peelle B et al (2001) Chem. Biol. 8:521-534); human cytotoxic T lymphocyte-associated antigen 4 (CTLA-4) (Hufton SE et al (2000) FEBS Lett. 475:225-231; Irving RA et al (2001) J. Immunol. Meth. 248:31-45); protease inhibitors, such as Knottin proteins (Wentzel A et al (2001) J. Bacteriol. 183:7273-7284; Baggio R et al (2002) J. Mol. Recognit. 15:126-134) and Kunitz domains (Roberts BL et al (1992) Gene 121:9-15; Dennis MS and Lazarus RA (1994) J. Biol. Chem. 269:22137-22144); PDZ domains (Schneider S et al (1999) Nat. Biotechnol. 17:170-175); peptide aptamers, such as thioredoxin (Lu Z et al (1995) Biotechnology 13:366-372; Klevenz B et al (2002) Cell. Mol. Life Sci. 59:1993-1998); staphylococcal nuclease (Norman TC et al (1999) Science 285:591-595); tendamistats (McConell SJ and Hoess RH (1995) J. Mol. Biol. 250:460-479; Li R et al (2003) Protein Eng. 16:65-72); trinectins based on the fibronectin type III domain (Koide A et al (1998) J. Mol. Biol. 284:1141-1151; Xu L et al (2002) Chem. Biol. 9:933-942); and zinc fingers (Bianchi E et al (1995) J. Mol. Biol. 247:154-160; Klug A (1999) J. Mol. Biol. 293:215-218; Segal DJ et al (2003) Biochemistry 42:2137-2148).

The above mentioned examples of non-immunoglobulin protein scaffolds include scaffold proteins presenting a single randomized loop used for the generation of novel binding specificities, protein scaffolds with a rigid secondary structure where side chains protruding from the protein surface are randomized for the generation of novel binding specificities, and scaffolds exhibiting a non-contiguous hyper-variable loop region used for the generation of novel binding specificities.

In addition to non-immunoglobulin proteins, oligonucleotides may also be used as affinity ligands. Single stranded nucleic acids, called aptamers or decoys, fold into well-defined three-dimensional structures and bind to their target with high affinity and specificity. (Ellington AD and Szostak JW (1990) Nature 346:818-822; Brody EN and Gold L (2000) J. Biotechnol. 74:5-13; Mayer G and Jenne A (2004) BioDrugs 18:351-359). The oligonucleotide ligands can be either RNA or DNA and can bind to a wide range of target molecule classes.

For selection of the desired affinity ligand from a pool of variants of any of the scaffold structures mentioned above, a number of selection platforms are available for the isolation of a specific novel ligand against a target protein of choice. Selection platforms include, but are not limited to, phage display (Smith GP (1985) Science 228:1315-1317), ribosome display (Hanes J and Plückthun A (1997) Proc. Natl. Acad. Sci. U.S.A. 94:4937-4942), yeast two-hybrid system (Fields S and Song O (1989) Nature 340:245-246), mRNA display (Roberts RW and Szostak JW (1997) Proc. Natl. Acad. Sci. U.S.A. 94:12297-12302), SELEX (System Evolution of Ligands by Exponential Enrichment) (Tuerk C and Gold L (1990) Science 249:505-510) and protein fragment complementation assays (PCA) (Remy I and Michnick SW (1999) Proc. Natl. Acad. Sci. U.S.A. 96:5394-5399).

Thus, in embodiments of the methods of the above aspects, an affinity ligand may be used, which is a non-immunoglobulin affinity ligand derived from any of the protein scaffolds listed above, or an oligonucleotide molecule.

In some embodiments of the methods of the above aspects, an affinity ligand capable of selective interaction with the CRABP2 protein is detectable and/or quantifiable. The detection and/or quantification of such an affinity ligand may be accomplished in any way known to the skilled person for detection and/or quantification of binding reagents in assays based on biological interactions. Thus, any affinity ligand, as described above, may be used quantitatively or qualitatively to detect the presence of the CRABP2 protein. These "primary" affinity ligands may be labeled themselves with various markers or are in turn detected by secondary, labeled affinity ligands to allow detection, visualization and/or quantification. This can be accomplished using any one or more of a multitude of labels, which can be conjugated to the affinity ligand capable of interaction with CRABP2 protein or to any secondary affinity ligand, using any one or more of a multitude of techniques known to the skilled person, and not as such involving any undue experimentation.

Non-limiting examples of labels that can be conjugated to primary and/or secondary affinity ligands include fluorescent dyes or metals (e.g. fluorescein, rhodamine, phycoerythrin, fluorescamine), chromophoric dyes (e.g. rhodopsin), chemiluminescent compounds (e.g. luminal, imidazole) and bioluminescent proteins (e.g. luciferin, luciferase), haptens (e.g. biotin). A variety of other useful fluorescers and chromophores are described in Stryer L (1968) Science 162:526-533 and Brand L and Gohlke JR (1972) Annu. Rev. Biochem. 41:843-868. Affinity ligands can also be labeled with enzymes (e.g. horseradish peroxidase, alkaline phosphatase, beta-lactamase), radioisotopes (e.g. ³H, ¹⁴C, ³²P, ³⁵S or ¹²⁵I) and particles (e.g. gold). Further, the affinity ligands may also be labeled with fluorescent semiconductor nanocrystals (Quantum dots). Quantum dots have superior quantum yield and are more photostable compared to organic fluorophores and are therefore more easily detected (Chan et al (2002) Curr Opi Biotech. 13: 40-46).The different types of labels can be conjugated to an affinity ligand using various chemistries, e.g. the amine reaction or the thiol reaction. However, other reactive groups than amines and thiols can be used, e.g. aldehydes, carboxylic acids and glutamine.

The method aspects above may be put to use in any of several known formats and set-ups, of which a non-limiting selection is discussed below.

In a set-up based on histology, the detection, localization and/or quantification of a labeled affinity ligand bound to its CRABP2 protein target may involve visual techniques, such as light microscopy or immunofluoresence microscopy. Other methods may involve the detection via flow cytometry or luminometry.

A biological sample, such as a tissue sample (biopsy), for example from breast tissue, may be removed from the subject for detection and/or quantification of CRABP2 protein. Alternatively, the biological sample, such as a biopsy, may be an earlier obtained sample. If using an earlier obtained sample, no steps of any one of the embodiments of the methods of the above aspects are practiced on the human or animal body. The affinity ligand may be applied to the biological sample for detection and/or quantification of the CRABP2 marker protein. This procedure enables not only detection of CRABP2 protein, but may in addition show the distribution and relative level of expression thereof.

The method of visualization of labels on the affinity ligand may include, but is not restricted to, fluorometric, luminometric and/or enzymatic techniques. Fluorescence is detected and/or quantified by exposing fluorescent labels to light of a specific wavelength and thereafter detecting and/or quantifying the emitted light in a specific wavelength region. The presence of a luminescently tagged affinity ligand may be detected and/or quantified by luminescence developed during a chemical reaction. Detection of an enzymatic reaction is due to a color shift in the sample arising from chemical reaction. Those of skill in the art are aware that a variety of different protocols can be modified in order for proper detection and/or quantification.

In embodiments of the methods of the above aspects, a biological sample may be immobilized onto a solid phase support or carrier, such as nitrocellulose or any other solid support matrix capable of immobilizing any CRABP2 protein present in the biological sample applied to it. Some well-known solid state support materials useful in the present invention include glass, carbohydrate (e.g. Sepharose), nylon, plastic, wool, polystyrene, polyethene, polypropylene, dextran, amylase, films, resins, cellulose, polyacrylamide, agarose, alumina, gabbros and magnetite. After immobilization of the biological sample, primary affinity ligand specific to CRABP2 may be applied, e.g. according to Examples, sections 2 and/or 3, of the present disclosure. If the primary affinity ligand is not labeled in itself, the supporting matrix may be washed with one or more appropriate buffers known in the art, followed by exposure to a secondary labeled affinity ligand and washed once again with buffers to remove unbound affinity ligands. Thereafter, selective affinity ligands may be detected and/or quantified with conventional methods. The binding properties for an affinity ligand may vary from one solid state support to the other, but those skilled in the art will be able to determine operative and optimal assay conditions for each determination by routine experimentation.

A method to detect and/or quantify the CRABP2 protein is by linking the affinity ligand to an enzyme that can then later be detected and/or quantified in an enzyme immunoassay (such as an EIA or ELISA). Such techniques are well established, and their realization does not present any undue difficulties to the skilled person. In such methods, the biological sample is brought into contact with a solid material or with a solid material conjugated to an affinity ligand against the CRABP2 protein, which is then detected and/or quantified with an enzymatically labeled secondary affinity ligand. Following this, an appropriate substrate is brought to react in appropriate buffers with the enzymatic label to produce a chemical moiety, which for example is detected and/or quantified using a spectrophotometer, fluorometer, luminometer or by visual means.

As stated above, primary and any secondary affinity ligands can be labeled with radioisotopes to enable detection and/or quantification. Non-limiting examples of appropriate radiolabels in the current invention are ³H, ¹⁴C, ³²P, ³⁵S or ¹²⁵I. The specific activity of the labeled affinity ligand is dependent upon the half-life of the radiolabel, isotopic purity, and how the label has been incorporated into the affinity ligand. Affinity ligands are preferably labeled using well-known techniques (Wensel TG and Meares CF (1983) in: Radioimmunoimaging and Radioimmunotherapy (Burchiel SW and Rhodes BA eds.) Elsevier, New York, pp 185-196). A thus radiolabeled affinity ligand can be used to visualize CRABP2 protein by detection of radioactivity *in vivo* or *in vitro.* Radionuclear scanning with e.g. gamma camera, magnetic resonance spectroscopy or emission tomography function for detection *in vivo* and *in vitro,* while gamma/beta counters, scintillation counters and radiographies are also used *in vitro.*

A further aspect of the present invention provides a kit for carrying out the methods according to the above aspects, which kit comprises:
a) a quantifiable affinity ligand capable of selective interaction with a CRABP2 protein; and
b) reagents necessary for quantifying the amount of the affinity ligand.

The various components of the kit according to the kit aspect are selected and specified as described above in connection with the method aspects of the present invention.

Thus, the kit according to the invention comprises an affinity ligand against CRABP2, as well as other means that help to quantify the specific and/or selective affinity ligand after it has bound specifically and/or selectively to CRABP2. For example, the kit may contain a secondary affinity ligand for detecting and/or quantifying a complex formed by any CRABP2 protein and the affinity ligand capable of selective interaction with a CRABP2 protein. The kit may also contain various auxiliary substances other than affinity ligands, to enable the kit to be used easily and efficiently. Examples of auxiliary substances include solvents for dissolving or reconstituting lyophilized protein components of the kit, wash buffers, substrates for measuring enzyme activity in cases where an enzyme is used as a label, target retrieval solution to enhance the accessibility to antigens in cases where paraffin or formalin-fixed tissue samples are used, and substances such as reaction arresters, e.g. endogenous enzyme block solution to decrease the background staining and/or counterstaining solution to increase staining contrast, that are commonly used in immunoassay reagent kits.

Thus, in embodiments of the kit aspect, the quantifiable affinity ligand is selected from the group consisting of antibodies, fragments thereof and derivatives thereof. Alternatively, the quantifiable affinity ligand is a protein ligand derived from a scaffold selected from the group consisting of staphylococcal protein A and domains thereof, lipocalins, ankyrin repeat domains, cellulose binding domains, γ crystallines, green fluorescent protein, human cytotoxic T lymphocyte-associated antigen 4, protease inhibitors, PDZ domains, peptide aptamers, staphylococcal nuclease, tendamistats, fibronectin type III domain and zinc fingers. As a further alternative, the quantifiable affinity ligand is an oligonucleotide molecule.

Further, in embodiments of the kit aspect, the detectable affinity ligand comprises a label selected from the group consisting of fluorescent dyes and metals, chromophoric dyes, chemiluminescent compounds and bioluminescent proteins, enzymes, radioisotopes, particles and quantum dots. Alternatively, the reagents necessary for quantifying the amount of the affinity ligand comprise a secondary affinity ligand capable of recognizing the quantifiable affinity ligand. As an example, the secondary affinity ligand capable of recognizing the quantifiable affinity ligand comprises a label selected from the group consisting of fluorescent dyes or metals, chromophoric dyes, chemiluminescent compounds and bioluminescent proteins, enzymes, radioisotopes, particles and quantum dots.

The kit may also advantageously comprise a reference sample for provision of, or yielding, the reference value to be used for comparison with the sample value. Such a reference sample may for example be constituted by a sample of tissue having a predetermined amount of CRABP2 protein, which may then be used by the person of skill in the art to determine the CRABP2 expression status in the sample being studied, by ocular or automated comparison of expression levels in the reference sample and the subject sample. For example, the reference sample may comprise cell lines, such as cancer cell lines, expressing a predetermined, or controlled, amount of CRABP2 protein. The person skilled in the art understands how to provide such cell lines, for example guided by the disclosure of Rhodes et al. (2006) The biomedical scientist, p 515-520. As an example, the cell lines may be formalin fixed. Also, the formalin fixed cell lines may be paraffin embedded.

The wording "reference sample for provision of the reference value" is to be interpreted broadly in the context of the kit aspect. The reference sample may contain an amount of CRABP2 protein actually corresponding to the reference value, but it may also contain an amount of CRABP2 protein corresponding to a value being higher than the reference value. In the latter case, the "high" value may be used by a person performing the method as an upper reference for assessing, e.g. the appearance of, a reference value which is lower than the "high" value. The skilled person understands how to do such an assessment. Further, the skilled person may for example use another reference sample comprising a low amount of CRABP2 protein for provision of a "low" value in such an assessment, e.g. as a negative reference.

Consequently, in embodiments of the kit aspect, the reference sample may comprise an amount of CRABP2 protein corresponding to a reference value. As an example, the reference sample may comprise an amount of CRABP2 protein corresponding to a nuclear fraction of 1 % or higher, such as 2 % or higher, such as 5 % or higher, such as 10 % or higher, such as 15 % or higher, such as 20 % or higher, such as 25 % or higher, such as 30 % or higher, such as 35 % or higher, such as 40 % or higher, such as 45 % or higher, such as 50 % or higher, such as 55 % or higher, such as 60 % or higher. Alternatively, or as a complement, the reference sample may comprise an amount of CRABP2 protein corresponding to an absent nuclear intensity of CRABP2 protein expression or higher, such as a weak nuclear intensity of CRABP2 protein expression or higher, such as moderate nuclear intensity of CRABP2 protein expression. Consequently, the reference sample may comprise an amount of CRABP2 protein corresponding to a nuclear fraction of 25 % or higher and a weak nuclear intensity of CRABP2 expression or higher. The provision of nuclear fraction values or nuclear intensity values is discussed above in connection with the method aspects.

Further, in alternative, or complementing, embodiments, the reference sample may comprise an amount of CRABP2 protein corresponding to a value being higher than the reference value. In these embodiments, the reference sample may for example comprise an amount of CRABP2 protein corresponding to a nuclear fraction of 75 % or higher and/or a strong nuclear intensity of CRABP2 expression.

Also, in alternative, or complementing, embodiments, the reference sample may comprise an amount of CRABP2 protein corresponding to a value being lower than the reference value.

As an alternative way of expressing the same idea, embodiments of the kit aspect may comprise a reference sample comprising an amount of CRABP2 protein corresponding to a predetermined reference value.

In alternative or complementing embodiments of the kit aspect, the kit may comprise a reference sample comprising an amount of CRABP2 protein corresponding to a value being higher than a predetermined reference value.

Further, in alternative or complementing embodiments of the kit aspect, the kit may comprise a reference sample comprising an amount of CRABP2 protein corresponding to a value being lower than a predetermined reference value.

Consequently, embodiments of the kit may comprise: a first reference sample comprising an amount of CRABP2 protein being higher than a predetermined reference value; and a second reference sample comprising an amount of CRABP2 protein being lower than the predetermined reference value.

In embodiments of the kit aspect, the reference sample may be a tissue sample, such as a tissue sample adapted to ocular or microscopic evaluation. As an example, the tissue reference sample may be fixated in paraffin or buffered formalin and/or histo-processed to µm-thin sections that are mounted on microscopic glass-slides. The tissue reference sample may be further adapted to staining with affinity ligands, such as antibodies, for a CRABP2 protein.

As a further aspect of the present invention, there is provided the use a CRABP2 protein as an endocrine treatment indicator for a mammalian subject having a breast cancer. In the context of the present disclosure, an "endocrine treatment indicator" refers to something that indicates a suitability of an endocrine treatment, such as a responsiveness to an endocrine treatment. As an example, the "endocrine treatment indicator for a mammalian subject having a breast cancer" may give information or guidance about whether the subject, such as a human, should undergo an endocrine treatment or is likely to benefit from an endocrine treatment.

As a related aspect of the present invention, there is provided the use of a CRABP2 protein, or an antigenically active fragment thereof, in the manufacture of an endocrine treatment indicator for a mammalian subject having a breast cancer. An antigenically active fragment of a CRABP2 protein is a fragment of sufficient size to be useful for the generation of an affinity ligand, e.g. an antibody, which will interact with a CRABP2 protein comprising the fragment. In this aspect, the endocrine treatment indicatormay be a CRABP2 protein comprising product that may be used for determining whether the subject, such as a human, should undergo an endocrine treatment or is likely to benefit from an endocrine treatment. However, the endocrine treatment indicatormay also be an affinity ligand, such as an antibody, with affinity for a CRABP2 protein, which endocrine treatment indicatormay be used for the same purposes.

In embodiments of the above use aspects, the breast cancer may be a hormone receptor negative breast cancer, such as an ER- or PR- breast cancer, an ER- breast cancer, a PR- breast cancer, or an ER- and PR- breast cancer.

In embodiments of the methods of the above aspects, the subject may be a human.

Further, in embodiments of the above use aspect, the subject may be a premenopausal female.

Also, in embodiments of the above use aspect, the breast cancer may be a TNM stage II (i.e. pT2 N0 M0, pT1 N1 M0 or pT2 N1 M0) breast carcinoma.

Further, in embodiments of the above use aspects, the endocrine treatment may be selected from a selective estrogen receptor modulator (SERM) treatment, an aromatase inhibitor treatment, and a steroidal estrogen receptor antagonist treatment. The SERM treatment may be a treatment with e.g. toremifene, raloxifene, droloxifene, arzoxifene or tamoxifene. The aromatase inhibitor treatment may be a treatment with e.g. anastrozole, letrozole och exemestane. Further, the steroidal estrogen receptor antagonist treatment may e.g. be a treatment with fulvestrant. As an example, the endocrine treatment may be a SERM treatment, such as a SERM treatment selected from treatments with toremifene, raloxifene, droloxifene, arzoxifene or tamoxifene. For example, in embodiments of the methods of the above aspects, the endocrine treatment may be a tamoxifen treatment.

In embodiments of the above use aspects of the invention, the CRABP2 protein may, as a non-limiting example, have an amino acid sequence which comprises a sequence selected from:
i) SEQ ID NO:1; and
ii) a sequence which is at least 85 % identical to SEQ ID NO:1.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

As another non-limiting example, the CRABP2 protein may have an amino acid sequence which comprises a sequence selected from:
i) SEQ ID NO:2; and
ii) a sequence which is at least 85 % identical to SEQ ID NO:2.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2.

In another aspect of the invention, there is provided an affinity ligand capable of selective interaction with a CRABP2 protein, which is an antibody or a fragment or a derivative thereof. Such an antibody, or fragment or derivative thereof, may for example be one that is obtainable by a process comprising a step of immunizing an animal with a protein whose amino acid sequence comprises the sequence SEQ ID NO:1. Processes for the production of antibodies or fragments or derivatives thereof against a given target are known in the art, and may be applied in connection with this aspect of the present invention. Any of those variants of the CRABP2 protein (SEQ ID NO:2) or the antigenically active fragment thereof (SEQ ID NO:1) that are discussed above may, of course, be used in such a process for generating an antibody or a fragment or derivative thereof. Also provided is the above affinity ligand for use as an endocrine treatment indicator for a mammalian subject having a breast cancer. As an example, affinity ligand may be comprised in an indicator product that may be used of determining whether the subject, such as a human, should undergo an endocrine treatment or is likely to benefit from an endocrine treatment

As a further aspect thereof, there is provided the use of the above affinity ligand as an endocrine treatment indicator for a mammalian subject having a breast cancer.

Also, as a further aspect thereof, there is provided the use of the above affinity ligand in the manufacture of an endocrine treatment indicator for a mammalian subject having a breast cancer.

With regard to the aspects relating to the above affinity ligand, or uses thereof, the endocrine treatment indicator, may be used for determining whether the subject, such as a human, should undergo an endocrine treatment or is likely to benefit from an endocrine treatment

In embodiments of aspects relating to the above affinity ligand and uses thereof, the breast cancer may be a hormone receptor negative breast cancer, such as an ER- or PR- breast cancer, an ER- breast cancer, a PR-breast cancer, or an ER- and PR- breast cancer.

In embodiments of such aspects, the subject may be a human.

Further, in embodiments of such aspects, the subject may be an premenopausal female.

Also, in embodiments of such aspects, the breast cancer may be a TNM stage II (i.e. pT2 N0 M0, pT1 N1 M0 or pT2 N1 M0) breast carcinoma.

Further, in embodiments of such aspects, the endocrine treatment may be selected from a selective estrogen receptor modulator (SERM) treatment, an aromatase inhibitor treatment, and a steroidal estrogen receptor antagonist treatment. The SERM treatment may be a treatment with e.g. toremifene, raloxifene, droloxifene, arzoxifene or tamoxifene. The aromatase inhibitor treatment may be a treatment with e.g. anastrozole, letrozole och exemestane. Further, the steroidal estrogen receptor antagonist treatment may be a treatment with fulvestrant. As an example, the endocrine treatment may be a SERM treatment, such as a SERM treatment selected from treatments with toremifene, raloxifene, droloxifene, arzoxifene or tamoxifene. For example, in embodiments of the methods of the above aspects, the endocrine treatment may be a tamoxifen treatment.

As a further aspect of the present invention, there is provided an endocrine treatment product for use in the treatment of a mammalian subject, such as a human, having a breast cancer, wherein said subject is diagnosed as hormone receptor negative and CRABP2 protein positive.

As a related aspect thereof, there is provided a use of an endocrine treatment product in the manufacture of a medicament for treatment of a mammalian subject, such as a human, having a breast cancer, wherein said subject is diagnosed as hormone receptor negative and CRABP2 protein positive.

The subject being diagnosed as "hormone receptor negative" refers to that the breast cancer from the subject has been found to be negative for a hormone receptor, such as ER or PR. The person skilled in the art knows how to determine whether a breast cancer is negative for a hormone receptor or not. For example, a commercially available kit for determining ER or PR status may be used for the determination. The commercially available kit may for example be ER/PR pharmDX (DakoCytomation) and the skilled person may use the kit according to the manufacturers instructions.

Further, for establishing an ER or PR diagnosis, the skilled person may turn to Allred et al (1998) Mod Pathol 11(2), 155. Allred presents a total score (Allred score), and, for both ER and PR, an Allred score of higher than two is considered positive and an Allred score of two or lower is considered negative.

Alternatively, when classifying a sample as being hormone receptor positive or negative, such as ER+ or ER-, the skilled person may use 10 % positive cells as the cutoff, which is a recognized limit within the art.

In embodiments of the endocrine treatment product aspects, the hormone receptor negative breast cancer may be an ER- or PR- breast cancer, such as an ER- breast cancer, a PR- breast cancer, or an ER- and PR- breast cancer.

The subject being diagnosed as "CRABP2 protein positive" refers to that a tumor of a subject has been found positive for CRABP2. For example, the subject may be considered to be diagnosed as CRABP2 protein positive if any CRABP2 protein parameter derived from said subject indicates that the subject is likely to benefit from an endocrine treatment. Further, the subject may be considered CRABP2 protein positive if a relevant biological sample from the subject has been found to contain an amount of CRABP2 protein corresponding to a sample value being higher than a relevant reference value. Relevant sample and reference values are discussed above in connection with the method aspects. As a non-limiting example, a breast cancer subject may be considered as CRABP2 protein positive if a relevant sample such as a primary or secondary tumor sample from the subject contains an amount of CRABP2 corresponding to nf >25 % or ni = 2-3 (moderate or strong).

In embodiments of the endocrine treatment product aspects, the endocrine treatment product may be selected from a selective estrogen receptor modulator (SERM), an aromatase, and a steroidal estrogen receptor antagonist. The SERM may be e.g. toremifene, raloxifene, droloxifene, arzoxifene or tamoxifene. The aromatase inhibitor may be e.g. anastrozole, letrozole och exemestane. Further, the steroidal estrogen receptor antagonist may be fulvestrant. As an example, the endocrine treatment product may be a SERM, such as a SERM selected from toremifene, raloxifene, droloxifene, arzoxifene or tamoxifene. For example, in embodiments of the endocrine treatment product aspects, the endocrine treatment may be tamoxifen.

Further, in embodiments of the endocrine treatment product aspects, the subject may be a premenopausal female.

Also, in embodiments of the endocrine treatment product aspects, the breast cancer may be a TNM stage II (i.e. pT2 N0 M0, pT1 N1 M0 or pT2 N1 M0) breast carcinoma.

### Brief description of the figures

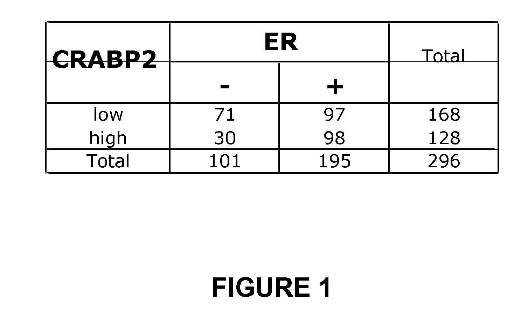

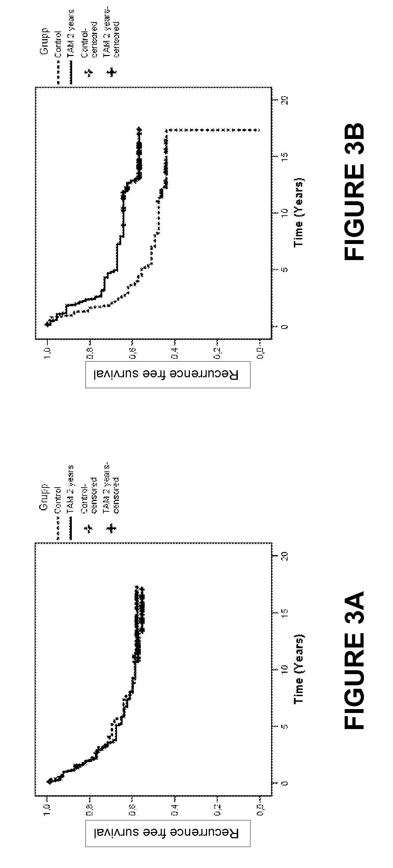

Figure 1 shows immunohistochemical staining levels of CRABP2 in 296 patients diagnosed with breast carcinoma. The subjects were scored for high or low CRABP2 level, wherein a high CRABP2 level is nf > 25% and/or ni = 2-3, and a low CRABP2 level is a nf <= 25% and ni = 0-1. Further, the subjects were classified as ER+ or ER-, wherein a fraction score of < 10 % was considered negative and a fraction score of > 10 % was considered positive.

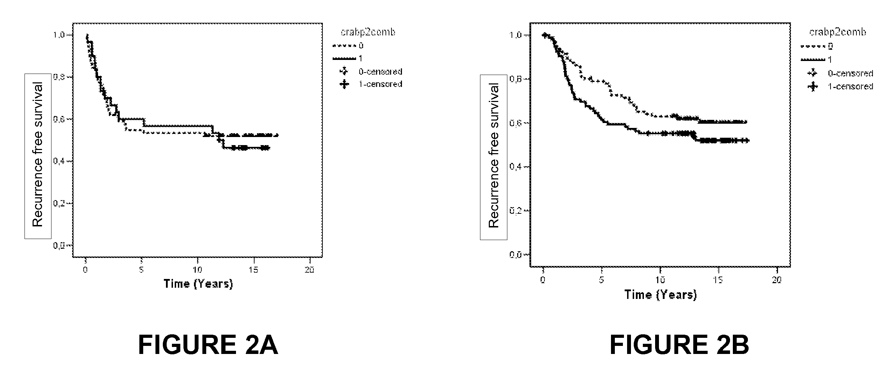

Figure 2 shows the results of a survival analysis based on immunohistochemical staining of 296 subjects diagnosed with breast carcinomas. Tissue cores were scored for high or low CRABP2 level, wherein a high CRABP2 level is nf > 25% and/or ni = 2-3 (represented by a solid line), and a low CRABP2 level is a nf <= 25% and ni = 0-1 (represented by a dotted line). Figure 2a shows recurrence free survival in ERα- patients. Figure 2b shows recurrence free survival in ERα+ patients.
   With regard to figures 3-12, tissue cores were scored for high or low CRABP2 level, wherein a high CRABP2 level is nf > 25% and/or ni = 2-3, and a low CRABP2 level is a nf <= 25% and ni = 0-1. Further, in figures 3-12, a solid line represents a group of subjects given adjuvant tamoxifen for 2 years, and a dotted line represents a control group not given tamoxifen.
Figure 3 shows the results of a survival analysis based on immunohistochemical staining of 296 subjects diagnosed with breast carcinoma. Figure 3a shows recurrence free survival in CRABP2 low patients. Figure 3b shows recurrence free survival in CRABP2 high patients.
Figure 4 shows the results of a survival analysis based on immunohistochemical staining of 195 subjects diagnosed with breast carcinoma. Figure 4a shows recurrence free survival in CRABP2 low and ERα+ patients. Figure 4b shows recurrence free survival in CRABP2 high and ERα+ patients.
Figure 5 shows the results of a survival analysis based on immunohistochemical staining of 101 subjects diagnosed with breast carcinoma. Figure 5a shows recurrence free survival in ERα- and CRABP2 low patients. Figure 5b shows recurrence free survival in ERα- and CRABP2 high patients.
Figure 6 shows the results of a survival analysis based on immunohistochemical staining of 101 subjects diagnosed with breast carcinoma. Figure 6a shows breast cancer specific survival in ERα- and CRABP2 low patients. Figure 6b shows breast cancer specific survival in ERα- and CRABP2 high patients.
Figure 7 shows the results of a survival analysis based on immunohistochemical staining of 100 subjects diagnosed with breast carcinomas. Figure 7a shows recurrence free survival in PR- and CRABP2 low patients. Figure 7b shows recurrence free survival in PR- and CRABP2 high patients.
Figure 8 shows the results of a survival analysis based on immunohistochemical staining of 100 subjects diagnosed with breast carcinomas. Figure 8a shows breast cancer specific survival in PR- and CRABP2 low patients. Figure 8b shows breast cancer specific survival in PR- and CRABP2 high patients.
Figure 9 shows the results of a survival analysis based on immunohistochemical staining of 112 subjects diagnosed with breast carcinomas. Figure 9a shows recurrence free survival in ER- and/or PR- and CRABP2 low patients. Figure 9b shows recurrence free survival in ER- and/or PR- and CRABP2 high patients.
Figure 10 shows the results of a survival analysis based on immunohistochemical staining of 112 subjects diagnosed with breast carcinomas. Figure 10a shows breast cancer specific survival in ER- and/or PR- and CRABP2 low patients. Figure 10b shows breast cancer specific survival in ER- and/or PR- and CRABP2 high patients.
Figure 11 shows the results of a survival analysis based on immunohistochemical staining of 89 subjects diagnosed with breast carcinomas. Figure 11 a shows recurrence free survival in ER-, PR- and CRABP2 low patients. Figure 11b shows recurrence free survival in ER-, PR- and CRABP2 high patients.
Figure 12 shows the results of a survival analysis based on immunohistochemical staining of 89 subjects diagnosed with breast carcinomas. Figure 12a shows breast cancer specific survival in ER-, PR- and CRABP2 low patients. Figure 12b shows breast cancer specific survival in ER-, PR- and CRABP2 high patients.

### Examples

### Generation of mono-specific antibodies against CRABP2 and use thereof to detect CRABP2 in normal and cancerous samples

### 1) Generation of antigen

### a. Materials and methods

A suitable fragment of the target protein encoded by the EnsEMBL Gene ID ENSG00000143320 was selected using bioinformatic tools with the human genome sequence as template (Lindskog M et al (2005) Biotechniques 38:723-727, EnsEMBL, www.ensembl.org). The fragment was used as template for the production of a 94 amino acid long fragment corresponding to amino acids 44-137 (SEQ ID NO:1) of the CRABP2 protein (SEQ ID NO:2; EnsEMBL entry no. ENSP00000357204). A polynucleotide encoding the target protein, which polynucleotide contained nucleotides 226-510 of the long CRABP2 gene transcript (SEQ ID NO:3; EnsEMBL entry no. ENST00000368221), was isolated by a Superscript^{™} One-Step RT-PCR amplification kit with Platinum® Taq (Invitrogen) and a human total RNA pool panel as template (Human Total RNA Panel IV, BD Biosciences Clontech). Flanking restriction sites Notl and Ascl were introduced into the fragment through the PCR amplification primers, to allow in-frame cloning into the expression vector (forward primer: ATCAAACAGGAGGGAGACAC, reverse primer: CTCTCGGACGTAGACCCTG). Then, the downstream primer was biotinylated to allow solid-phase cloning as previously described, and the resulting biotinylated PCR product was immobilized onto Dynabeads M280 Streptavidin (Dynal Biotech) (Larsson M et al (2000) J. Biotechnol. 80:143-157). The fragment was released from the solid support by Notl-Ascl digestion (New England Biolabs), ligated into the pAff8c vector (Larsson M *et al*, *supra)* in frame with a dual affinity tag consisting of a hexahistidyl tag for immobilized metal ion chromatography (IMAC) purification and an immunopotentiating albumin binding protein (ABP) from streptococcal protein G (Sjölander A et al (1997) J. Immunol. Methods 201:115-123; Ståhl S et al (1999) Encyclopedia of Bioprocess Technology: Fermentation, Biocatalysis and Bioseparation (Fleckinger MC and Drew SW, eds) John Wiley and Sons Inc., New York, pp 49-63), and transformed into *E. coli* BL21(DE3) cells (Novagen). The sequences of the clones were verified by dye-terminator cycle sequencing of plasmid DNA amplified using TempliPhi DNA sequencing amplification kit (GE Healthcare, Uppsala, Sweden) according to the manufacturer's recommendations.

BL21 (DE3) cells harboring the expression vector were inoculated in 100 ml 30 g/l tryptic soy broth (Merck KGaA) supplemented with 5 g/l yeast extract (Merck KGaA) and 50 mg/l kanamycin (Sigma-Aldrich) by addition of 1 ml of an overnight culture in the same culture medium. The cell culture was incubated in a 1 liter shake flask at 37 °C and 150 rpm until the optical density at 600 nm reached 0.5-1.5. Protein expression was then induced by addition of isopropyl-β-D-thiogalactopyranoside (Apollo Scientific) to a final concentration of 1 mM, and the incubation was continued overnight at 25 °C and 150 rpm. The cells were harvested by centrifugation at 2400 *g*, and the pellet was re-suspended in 5 ml lysis buffer (7 M guanidine hydrochloride, 47 mM Na₂HPO₄, 2.65 mM NaH₂PO₄, 10 mM Tris-HCl, 100 mM NaCl, 20 mM β-mercaptoethanol; pH = 8.0) and incubated for 2 hours at 37 °C and 150 rpm. After centrifugation at 35300 g, the supernatant containing the denatured and solubilized protein was collected.

The His₆-tagged fusion protein was purified by immobilized metal ion affinity chromatography (IMAC) on columns with 1 ml Talon® metal (Co²⁺) affinity resin (BD Biosciences Clontech) using an automated protein purification procedure (Steen J et al (2006) Protein Expr. Purif. 46:173-178) on an ASPEC XL4™ (Gilson). The resin was equilibrated with 20 ml denaturing washing buffer (6 M guanidine hydrochloride, 46.6 mM Na₂HPO₄, 3.4 mM NaH₂PO₄, 300 mM NaCl, pH 8.0-8.2). Clarified cell lysates were then added to the column. Thereafter, the resin was then washed with a minimum of 31.5 ml washing buffer prior to elution in 2.5 ml elution buffer (6 M urea, 50 mM NaH₂PO₄, 100 mM NaCl, 30 mM acetic acid, 70 mM Na-acetate, pH 5.0). The eluted material was fractioned in three pools of 500, 700 and 1300 µl. The 700 µl fraction, containing the antigen, and the pooled 500 and 1300 µl fractions were stored for further use.

The antigen fraction was diluted to a final concentration of 1 M urea with phosphate buffered saline (PBS; 1.9 mM NaH₂PO₄, 8.1 mM Na₂HPO₄, 154 mM NaCl) followed by a concentration step to increase the protein concentration using Vivapore 10/20 ml concentrator with molecular weight cut off at 7500 Da (Vivascience AG). The protein concentration was determined using a bicinchoninic acid (BCA) micro assay protocol (Pierce) with a bovine serum albumin standard according to the manufacturer's recommendations. The protein quality was analyzed on a Bioanalyzer instrument using the Protein 50 or 200 assay (Agilent Technologies).

### b) Results

A gene fragment corresponding to nucleotides 226-510 of the long transcript (SEQ ID NO:3) of the CRABP2 gene and encoding a peptide (SEQ ID NO:1) consisting of amino acids 44 to 137 of the target protein CRABP2 (SEQ ID NO:2) was successfully isolated by RT-PCR from a human RNA pool using primers specific for the protein fragment. The 94 amino acid fragment (SEQ ID NO:1) of the target protein (SEQ ID NO:2) was designed to lack transmembrane regions to ensure efficient expression in *E. coli*, and to lack any signal peptide, since those are cleaved off in the mature protein. In addition, the protein fragment was designed to consist of a unique sequence with low homology with other human proteins, to minimize cross reactivity of generated affinity reagents, and to be of a suitable size to allow the formation of conformational epitopes and still allow efficient cloning and expression in bacterial systems.

A clone encoding the correct amino acid sequence was identified, and, upon expression in *E. coli*, a single protein of the correct size was produced and subsequently purified using immobilized metal ion chromatography. After dilution of the eluted sample to a final concentration of 1 M urea and concentration of the sample to 1 ml, the concentration of the protein fragment was determined to be 6.047 mg/ml and was 83.4 % pure according to purity analysis.

### 2) Generation of antibodies

### a) Materials and methods

The purified CRABP2 fragment as obtained above was used as antigen to immunize a rabbit in accordance with the national guidelines (Swedish permit no. A 84-02). The rabbit was immunized intramuscularly with 200 µg of antigen in Freund's complete adjuvant as the primary immunization, and boosted three times in four week intervals with 100 µg antigen in Freund's incomplete adjuvant.

Antiserum from the immunized animal was purified by a three-step immunoaffinity based protocol (Agaton C et al (2004) J. Chromatogr. A 1043:33-40; Nilsson P et al (2005) Proteomics 5:4327-4337). In the first step, 7 ml of total antiserum was buffered with 10x PBS to a final concentration of 1x PBS (1.9 mM NaH₂PO₄, 8.1 mM Na₂HPO₄, 154 mM NaCl), filtered using a 0.45 µm pore-size filter (Acrodisc®, Life Science) and applied to an affinity column containing 5 ml N-hydroxysuccinimide-activated Sepharose^{™} 4 Fast Flow (GE Healthcare) coupled to the dual affinity tag protein His₆-ABP (a hexahistidyl tag and an albumin binding protein tag) expressed from the pAff8c vector and purified in the same way as described above for the antigen protein fragment. In the second step, the flow-through, depleted of antibodies against the dual affinity tag His₆-ABP, was loaded at a flow rate of 0.5 ml/min on a 1 ml Hi-Trap NHS-activated HP column (GE Healthcare) coupled to the CRABP2 protein fragment used as antigen for immunization (SEQ ID NO:1). The His₆-ABP protein and the protein fragment antigen had been coupled to the NHS activated matrix as recommended by the manufacturer. Unbound material was washed away with 1 x PBST (1 x PBS, 0.1 % Tween20, pH 7.25), and captured antibodies were eluted using a low pH glycine buffer (0.2 M glycine, 1 mM EGTA, pH 2.5). The eluted antibody fraction was collected automatically, and loaded onto two 5 ml HiTrap™ desalting columns (GE Healthcare) connected in series for efficient buffer exchange in the third step. The second and third purification steps were run on the ÄKTAxpress™ platform (GE Healthcare). The antigen selective (mono-specific) antibodies (msAbs) were eluted with PBS buffer, supplemented with glycerol and NaN₃ to final concentrations of 40 % and 0.02 %, respectively, for long term storage at -20 °C (Nilsson P et al (2005) Proteomics 5:4327-4337).

The specificity and selectivity of the affinity purified antibody fraction were analyzed by binding analysis against the antigen itself and against 94 other human protein fragments in a protein array set-up (Nilsson P et al (2005) Proteomics 5:4327-4337). The protein fragments were diluted to 40 µg/ml in 0.1 M urea and 1x PBS (pH 7.4) and 50 µl of each were transferred to the wells of a 96-well spotting plate. The protein fragments were spotted and immobilized onto epoxy slides (SuperEpoxy, TeleChem) using a pin-and-ring arrayer (Affymetrix 427). The slide was washed in 1x PBS (5 min) and the surface was then blocked (SuperBlock®, Pierce) for 30 minutes. An adhesive 16-well silicone mask (Schleicher & Schuell) was applied to the glass before the mono-specific antibodies were added (diluted 1:2000 in 1x PBST to appr. 50 ng/ml) and incubated on a shaker for 60 min. Affinity tag-specific IgY antibodies were co-incubated with the mono-specific antibodies in order to quantify the amount of protein in each spot. The slide was washed with 1x PBST and 1x PBS twice for 10 min each. Secondary antibodies (goat anti-rabbit antibody conjugated with Alexa 647 and goat anti-chicken antibody conjugated with Alexa 555, Molecular Probes) were diluted 1:60000 to 30 ng/ml in 1x PBST and incubated for 60 min. After the same washing procedure, as for the first incubation, the slide was spun dry and scanned (G2565BA array scanner, Agilent), thereafter images were quantified using image analysis software (GenePix 5.1, Axon Instruments).

In addition, the specificity and selectivity of the affinity-purified antibody were analyzed by Western blot. In addition, the specificity and selectivity of the affinity-purified antibody were analyzed by Western blot. Western blot was performed by separation of total protein extracts from selected human cell lines on pre-cast 10-20 % SDS-PAGE gradient gels (Bio-Rad Laboratories) under reducing conditions, followed by electro-transfer to PVDF membranes (Bio-Rad Laboratories) according to the manufacturer's recommendations. The membranes were blocked (5 % dry milk, 1x TBST; 0.1 M Tris-HCl, 0.5 M NaCl, 0.1 % Tween20) for 1 h at room temperature, incubated with the primary affinity purified antibody (diluted 1:500 in blocking buffer) and washed in TBST. The secondary HRP-conjugated antibody (swine anti-rabbit immunoglobulin/HRP, DakoCytomation) was diluted 1:3000 in blocking buffer and chemiluminescence detection was carried out using a Chemidoc^{™} CCD camera (Bio-Rad Laboratories) and SuperSignal® West Dura Extended Duration substrate (Pierce), according to the manufacturer's protocol.

### b) Results

The quality of polyclonal antibody preparations has proven to be dependent on the degree of stringency in the antibody purifications, and it has previously been shown that depletion of antibodies directed against epitopes not originated from the target protein is necessary to avoid cross-reactivity to other proteins and background binding (Agaton C et al (2004) J. Chromatogr. A 1043:33-40).

Thus, a protein microarray analysis was performed to ensure that mono-specific polyclonal antibodies of high specificity had been generated by depletion of antibodies directed against the His₆-tag as well as of antibodies against the ABP-tag. This was followed by affinity capture of antigen selective antibodies on an affinity column with immobilized antigen.

To quantify the amount of protein in each spot of the protein array, a two-color dye labeling system was used, with a combination of primary and secondary antibodies. Tag-specific IgY antibodies generated in hen were detected with a secondary goat anti-hen antibody labeled with Alexa 555 fluorescent dye. The specific binding of the rabbit msAb to its antigen on the array was detected with a fluorescently Alexa 647 labeled goat anti-rabbit antibody. Each protein fragment was spotted in duplicates. The protein array analysis shows that the affinity purified mono-specific antibody against CRABP2 is highly selective to the correct protein fragment and has a very low background to all other protein fragments analyzed on the array.

The result of the Western blot analysis shows that the antibody specifically detects a single band of approximately 16 kDa in two breast tumor cell lines, T47D and MCF-7. The theoretical molecular weight of CRABP2 is 16 kDa (as calculated from the CRABP2 amino acid sequence SEQ ID NO:2), corresponding well to the result obtained.

### 3) Tissue profiling by immunohistochemistry

### a) Material and Methods

In total, 576 paraffin cores containing human tissues were analyzed using the mono-specific antibody sample. All tissues used as donor blocks for tissue microarray (TMA) production were selected from the archives at the Department of Pathology, University Hospital, Uppsala, in agreement with approval from the local ethical committee. Corresponding tissue sections were examined to determine diagnosis and to select representative areas in donor blocks. Normal tissue was defined as microscopically normal (non-neoplastic) and was most often selected from specimens collected from the vicinity of surgically removed tumors. Cancer tissue was reviewed for diagnosis and classification. All tissues were formalin fixated, paraffin embedded, and sectioned for diagnostic purposes.

The TMA production was performed essentially as previously described (Kononen J et al (1998) Nature Med. 4:844-847; Kallioniemi OP et al (2001) Hum. Mol. Genet. 10:657-662). Briefly, a hole was made in the recipient TMA block and a cylindrical core tissue sample from the donor block was acquired and deposited in the recipient TMA block. This was repeated in an automated tissue arrayer from Beecher Instrument (ATA-27, Beecher Instruments, Sun Prairie, CA, USA) until a complete TMA design was produced. TMA recipient blocks were baked at 42 °C for 2 h prior to sectioning.

The design of TMA:s was focused on obtaining samples from a large range of representative normal tissues, and on including representative cancer tissues. This has previously been described in detail in Kampf C et al (2004) Clin. Proteomics 1:285-300. In brief, samples from 48 normal tissues and from 20 of the most common cancer types affecting humans were selected. In total, eight different designs of TMA blocks, each containing 72 cores of tissue with 1 mm diameter, were produced. Two of the TMA:s represented normal tissues, corresponding to 48 different normal tissues in triplicates from different individuals. The remaining 6 TMA:s represented cancer tissue from 20 different types of cancer. For 17 of the 20 cancer types, 12 individually different tumors were sampled, and for the remaining 3 cancer types, 4 individually different tumors were sampled, all in duplicates from the same tumor. The TMA blocks were sectioned with 4 µm thickness using a waterfall microtome (Leica), and placed onto SuperFrost® (Roche Applied Science) glass slides for immunohistochemical analysis.

Automated immunohistochemistry was performed as previously described (Kampf C et al (2004) Clin. Proteomics 1:285-300). In brief, the glass slides were incubated for 45 min in 60 °C, de-paraffinized in xylene (2 x 15 min) and hydrated in graded alcohols. For antigen retrieval, slides were immersed in TRS (Target Retrieval Solution, pH 6.0, DakoCytomation) and boiled for 4 min at 125 °C in a Decloaking chamber® (Biocare Medical). Slides were placed in the Autostainer® (DakoCytomation) and endogenous peroxidase was initially blocked with H₂O₂ (DakoCytomation). The primary antibody obtained as in Examples, Section 2 and goat anti-rabbit peroxidase conjugated Envision® were incubated for 30 min each at room temperature. Between all steps, slides were rinsed in wash buffer (DakoCytomation). Finally, diaminobenzidine (DakoCytomation) was used as chromogen and Harris hematoxylin (Sigma-Aldrich) was used for counterstaining. The slides were mounted with Pertex® (Histolab).

All immunohistochemically stained sections from the eight different TMA:s were scanned using a ScanScope T2 automated slide-scanning systems (Aperio Technologies). In order to represent the total content of the eight TMA:s, 576 digital images were generated. Scanning was performed at 20 times magnification. Digital images were separated and extracted as individual tagged image file format (TIFF) files for storage of original data. In order to be able to handle the images in a web-based annotation system, the individual images were compressed from TIFF format into JPEG format. All images of immunohistochemically stained tissue were manually evaluated under the microscope and annotated by a board certified pathologist or by specially educated personnel followed by verification of a pathologist.

Annotation of each different normal and cancer tissue was performed using a simplified scheme for classification of immunohistochemical outcome. Each tissue was examined for representativity and immunoreactivity. The different tissue specific cell types included in each normal tissue type were annotated. For each cancer, tumor cells and stroma were annotated. Basic annotation parameters included an evaluation of i) subcellular localization (nuclear and/or cytoplasmic/membranous), ii) staining intensity and iii) fraction of stained cells. Staining intensity was subjectively evaluated in accordance to standards used in clinical histo-pathological diagnostics and outcome was classified as: absent = no immunoreactivity, weak = faint immunoreactivity, moderate = medium immunoreactivity or strong = distinct and strong immunoreactivity. The fraction of stained cells was estimated and classified as < 2 %, 2-25 %, 26-75 % or > 75 % immunoreactive cells of the relevant cell population. Based on both the intensity and fraction of immunoreactive cells, a "staining score" was given for each tissue sample: 0 = negative, 1 = weak, 2 = moderate and 3 = strong. N.R. means that no representative tissues were present. The skilled artisan will recognize that this procedure is similar to a calculation of an Allred score, see e.g. Allred et al (1998) Mod Pathol 11 (2), 155.

### b) Results

The results from tissue profiling with the mono-specific antibody generated towards a recombinant protein fragment of the human target protein CRABP2 obtained as in Examples, Section 2 showed a particular immunoreactivity in several normal tissues (Table 1) and cancer tissues. Immunoreactivity was mainly observed in cytoplasm but also in nuclei.

Table 1 shows the CRABP2 protein expression pattern in normal human tissues. Using immunohistochemistry and TMA technology, 144 spots (1 mm in diameter) representing 48 different types of normal tissue were screened for expression of CRABP2. Strong expression (staining score 3) was found in adnexal skin cells and surface epithelial cells of vagina and vulva/analskin. Moderate (staining score 2) levels of expression were detected in glandular cells of breast, surface epithelial cells of esophagus, oral mucosa and urinary bladder and mesenchymal cells of soft tissue. All other cells and tissues had weak or no (staining score 1 or 0) expression. In a few cases no representative tissue (N.R.) were observed.

| **Table 1:** Expression pattern of CRABP2 in normal tissues | | |
|---|---|---|
| **Tissue type** | **Cell type** | **Staining score** |
| **Adrenal gland** | cortical cells | 0 |
| | medullar cells | N.R. |
| **Appendix** | glandular cells | 1 |
| | lymphoid tissue | 0 |
| **Bone marrow** | bone marrow poetic cells | 0 |
| **Breast** | glandular cells | 2 |
| **Bronchus** | surface epithelial cells | 1 |
| **Cerebellum** | cells in granular layer | 0 |
| | cells in molecular layer | 0 |
| | purkinje cells | 0 |
| **Cerebral cortex** | neuronal cells | 1 |
| | non-neuronal cells | 0 |
| **Cervix, uterine** | glandular cells | 0 |
| | surface epithelial cells (squamous) | 2 |
| **Colon** | glandular cells | 0 |
| **Duodenum** | glandular cells | 0 |
| **Endometrium 1** | cells in endometrial stroma/ECM | 0 |
| | cells in myometrium/ECM | 1 |
| | glandular cells | 0 |
| **Endometrium 2** | cells in endometrial stroma/ECM | 2 |
| | cells in myometrium/ECM | N.R. |
| | glandular cells | 0 |
| **Epididymis** | glandular cells | 1 |
| **Esophagus** | surface epithelial cells | 2 |
| **Fallopian tube** | glandular cells | 0 |
| **Gall bladder** | glandular cells | 0 |
| **Heart muscle** | myocytes | 0 |
| **Hippocampus** | neuronal cells | 1 |
| | non-neuronal cells | 0 |
| **Kidney** | cells in glomeruli | 0 |
| | cells in tubuli | 0 |
| **Lateral ventricle** | neuronal cells | 1 |
| | non-neuronal cells | 0 |
| **Liver** | bile duct cells | 0 |
| | hepatocytes | 0 |
| **Lung** | alveolar cells | 0 |
| | macrophages | 0 |
| **Lymph node** | follicle cells (cortex) | 0 |
| | non-follicle cells (paracortex) | 0 |
| **Nasopharynx** | surface epithelial cells | 1 |
| **Oral mucosa** | surface epithelial cells | 2 |
| **Ovary** | follicle cells | N.R. |
| | ovarian stromal cells | 0 |
| **Pancreas** | exocrine pancreas | 0 |
| | islet cells | 0 |
| **Parathyroid gland** | glandular cells | 0 |
| **Placenta** | decidual cells | N.R. |
| | trophoblastic cells | 1 |
| **Prostate** | glandular cells | 1 |
| **Rectum** | glandular cells | 0 |
| **Salivary gland** | glandular cells | 1 |
| **Seminal vescicle** | glandular cells | 1 |
| **Skeletal muscle** | myocytes | 0 |
| **Skin** | adnexal cells | 3 |
| | epidermal cells | 1 |
| **Small intestine** | glandular cells | 0 |
| **Smooth muscle** | smooth muscle cells | 0 |
| **Soft tissue 1** | mesenchymal cells | 2 |
| **Soft tissue 2** | mesenchymal cells | 2 |
| **Spleen** | cells in red pulp | 0 |
| | cells in white pulp | 0 |
| **Stomach 1** | glandular cells | 1 |
| **Stomach 2** | glandular cells | 1 |
| **Testis** | cells in ductus seminiferus | 1 |
| | leydig cells | 0 |
| **Thyroid gland** | glandular cells | 0 |
| **Tonsil** | follicle cells (cortex) | 0 |
| | non-follicle cells (paracortex) | 0 |
| | surface epithelial cells | 0 |
| **Urinary bladder** | surface epithelial cells | 2 |
| **Vagina** | surface epithelial cells | 3 |
| **Vulva/anal skin** | surface epithelial cells | 3 |

CRABP2 protein expression was evaluated in tissue samples from various cancer types. Table 2 shows the level of CRABP2 expression in 12 different breast carcinoma tissues samples. Nine of these samples showed representative tissue of which all showed positivity and seven 7 strong expression.

| **Table 2:** Expression pattern of CRABP2 | | | | | | | | | | | | |
|---|---|---|---|---|---|---|---|---|---|---|---|---|
| Tissue sample | 1 | 2 | 3 | 4 | 5 | 6 | 7 | 8 | 9 | 10 | 11 | 12 |
| Staining score | 3 | 3 | 3 | 3 | 3 | 3 | 3 | 2 | 1 | N.R. | N.R. | N.R. |

### 4) Breast cancer TMA

### a) Material and methods

Immunohistochemical analyses were performed on tissue microarrays representing 500 tumors from 564 premenopausal patients enrolled in a controlled, randomized tamoxifen trial of stage II (pT2 N0 M0, pT1 N1 M0 or pT2 N1 M0) primary breast carcinoma. The patients had been included in the trial between 1986-1991 irrespective of hormone receptor status and 276 patients had been allocated to two years of adjuvant tamoxifen and 288 to control. Median age at diagnosis was 45 (25-57) years. Median follow-up was 13.9 years and less than 2% of the patients had received adjuvant chemotherapy. The study design is described in detail elsewhere (Rydén et al, Eur J Cancer, 2005, 41 (2): 256-64). Ethical permission was obtained from the Local Ethics Committees at Lund and Linköping Universities.

All cases had been histopathologically re-evaluated on hematoxylin and eosin stained slides. TMA:s were then constructed by sampling 2 x 0.6 mm cores per case from areas representative of invasive cancer. The TMA:s were prepared and automated immunohistochemistry was performed as described in section 3 above, using the CRABP2 antibody prepared as described in section 2 above.

For statistical analyses, categories were based on nuclear fraction (nf), corresponding to the percentage of tumor cells in a sample that exhibits a positive staining in the nucleus, wherein a medium or distinct and strong immunoreactivity in the nucleus is considered positive and no or faint immunoreactivity is considered negative, and nuclear intensity (ni), corresponding to the over-all staining intensity of tumor cells in a sample. As with "staining intensity" in Examples, Section 3 above, ni was subjectively evaluated in accordance to standards used in clinical histo-pathological diagnostics and outcome was classified as: absent (0) = no immunoreactivity, weak (1) = faint immunoreactivity, moderate (2) = medium immunoreactivity or strong (3) = distinct and strong immunoreactivity. Further, as with "the fraction of stained cells" in Examples, Section 3 above, nf was classified as < 2 %, 2-25 %, 26-75 % or > 75 %. Two categories were defined: "CRABP2 low" corresponding to nf <= 25% and ni = 0-1; and "CRABP2 high" corresponding to nf > 25% and/or ni = 2-3 (Fig 1). This classification of samples was used for recurrence free survival (RFS) or breast cancer specific survival (BCSS) analysis according to the Kaplan-Meier estimator, and the log-rank test was used to compare survival in different strata. All statistical tests were two-sided, and p-values of < 0.05 % were considered significant. All calculations were made with the statistical package SPSS 12.0 (SPSS Inc. Illinois, USA).

### b) Results

For the present study, immunohistochemical analysis of CRAPB2 expression could be performed on 296 tumors. The remaining cores either did not contain invasive cancer or had been lost during histoprocessing. Of the 296 analyzed tumours, 195 were ERα+, defined as >10% ERα+ nuclear fraction, which is in line with the clinically established cut-off used for hormone receptor assessment. Analyses of recurrence free survival (RFS) based on low or high CRABP2 protein levels in ER negative (i.e. ERα-) and ER positive (i.e. ERα+) subjects are presented in fig 2A and 2B, respectively. From these analyses, a slightly better trend is observed for the CRABP2 low category than for the CRABP2 high category of ER positive subjects.

If the parameters were changed, and the RFS analysis was based on tamoxifen treatment of CRABP2 high or low subjects, irrespective of ER status, the results revealed an improved survival upon tamoxifen treatment for patients with CRABP2 high tumours (Fig 3B) in contrast to patients with CRABP2 low tumors, where tamoxifen treatment had no impact on survival (Fig 3A).

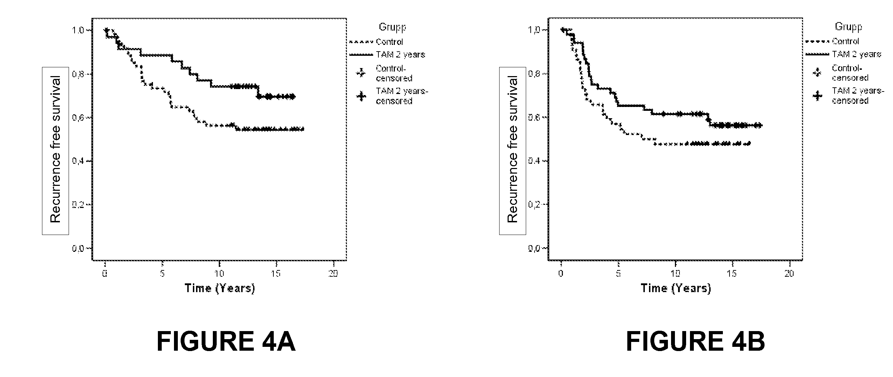

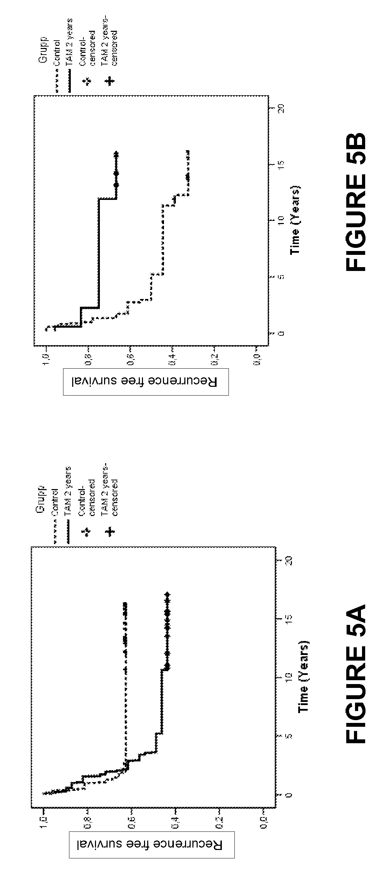

When analyzing the ERα+ tumors only, a trend towards a positive response to tamoxifen treatment were observed for both the CRABP2 low category and the CRABP2 high category (Fig 4A and 4B). However, when analyzing RFS in ERα- subjects only, the trend of CRABP2 low category that did not receive tamoxifen was better than for the CRABP2 low category that did receive tamoxifen treatment (Fig 5A). Consequently, tamoxifen treatment does not seem to be suitable for this group of subjects. In contrast, for patients with ERα- and CRABP2 high tumors, the order was reversed: the CRABP2 high category that received tamoxifen showed a better survival probability trend, such as a likelihood of five-year, ten-year or 15-year survival, than the control group (Fig 5B). In other words, the CRABP2 low category of ERα- subjects seems to be negatively affected by tamoxifen treatment, whereas the CRABP2 high category of ERα- subjects seems to be positively affected by tamoxifen treatment (Fig 5A and 5B).

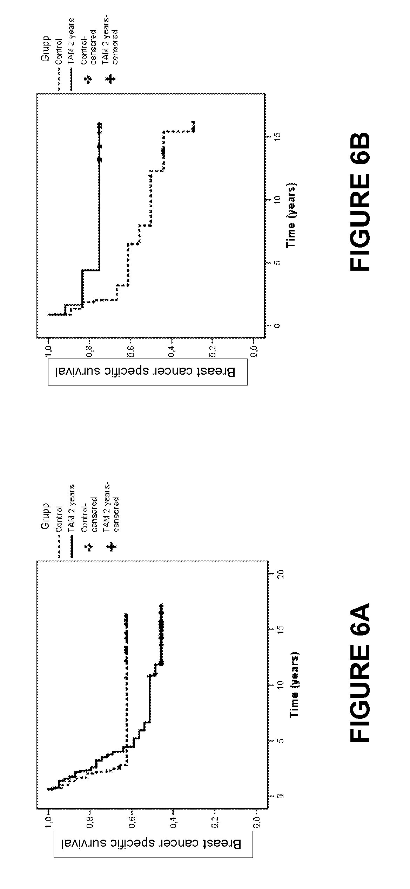

Similar trends were observed when analyzing BCSS in ERα- subjects only: the CRABP2 low category that received tamoxifen showed a worse trend than the CRABP2 low category that not received tamoxifen; and the CRABP2 high category that received tamoxifen showed a better trend than the CRABP2 low category that not received tamoxifen (Fig 6A and 6B).

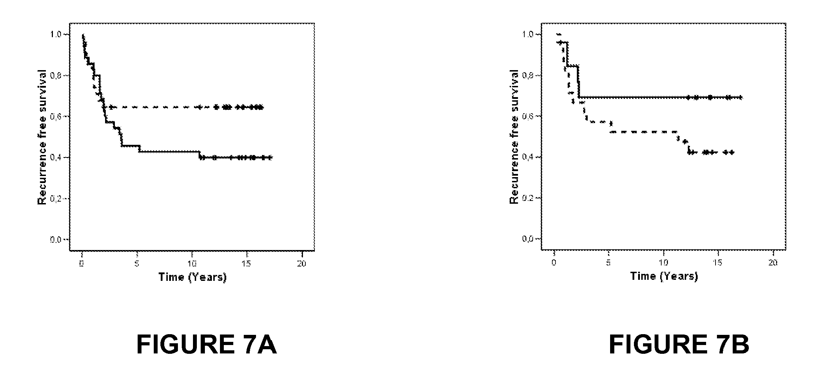

As with the group of ERα- subjects, a group of PR- subjects showed a positive response to tamoxifen treatment (Fig 7B) when analyzing RFS. The CRABP2 high category that received tamoxifen showed a better survival probability trend, such as a likelihood of five-year, ten-year or 15-year survival, than the control group (Fig 7B). Further, the CRABP2 low category of PR- subjects seems to be negatively affected by tamoxifen treatment (Fig 7A).

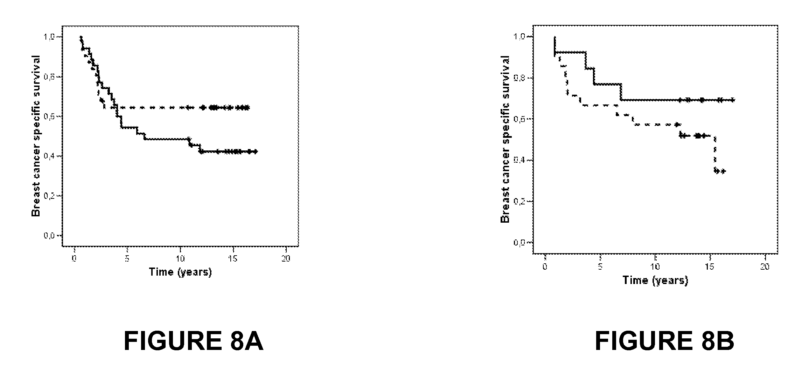

Similar trends were observed when analyzing BCSS in PR- subjects only: the CRABP2 low category that received tamoxifen showed a worse trend than the CRABP2 low category that not received tamoxifen; and the CRABP2 high category that received tamoxifen showed a better trend than the CRABP2 low category that not received tamoxifen (Fig 8A and 8B).

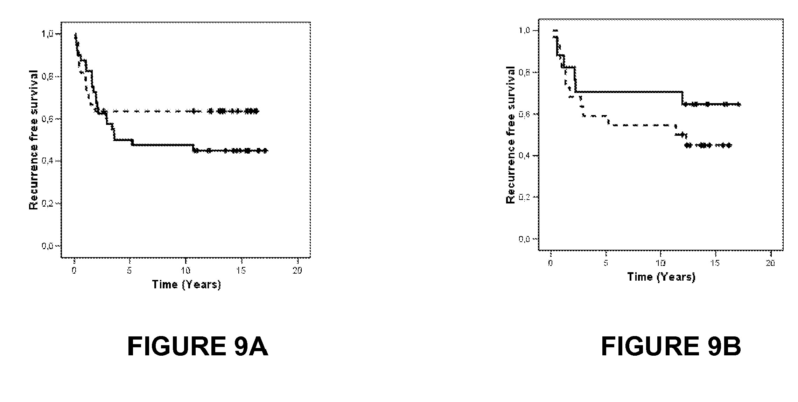

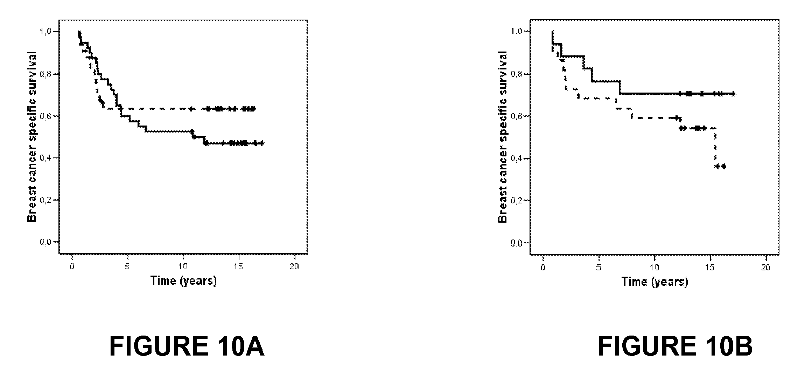

When a group of subjects being hormone receptor negative, i.e. ERα- and/or PR-, was analyzed, the same trends as with the ERα- group or PR-group taken individually were observed. The CRABP2 low category that received tamoxifen show a worse trend with regard to both RFS (Fig 9a) and BCSS (Fig 10a) than the CRABP2 low category that not received tamoxifen, and the CRABP2 high category that received tamoxifen show a better trend with regard to both RFS (Fig 9b) and BCSS (Fig 10b) than the CRABP2 low category that not received tamoxifen.

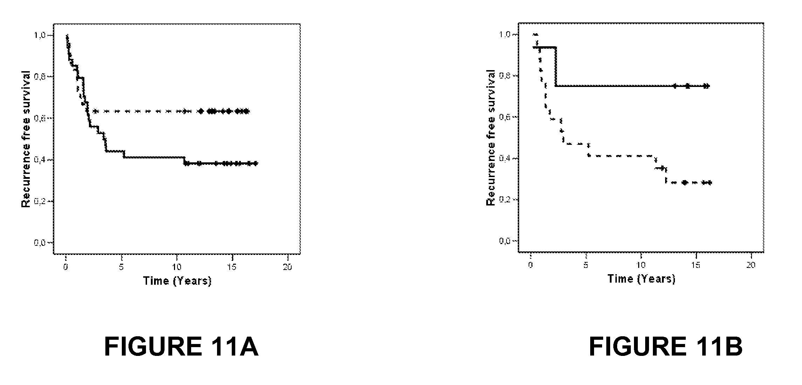

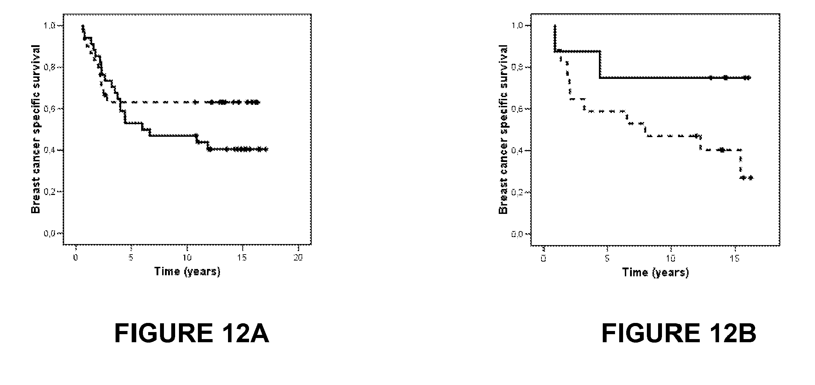

Finally, the results from an analysis of a group of subjects being negative for both ER and PR (ERα- and PR-) are consistent with those above. The CRABP2 low category that received tamoxifen show a worse trend with regard to both RFS (Fig 11a) and BCSS (Fig 12a) than the CRABP2 low category that not received tamoxifen, and the CRABP2 high category that received tamoxifen show a better trend with regard to both RFS (Fig 11b) and BCSS (Fig 12b) than the CRABP2 low category that not received tamoxifen.

### Determination of whether a breast cancer patient is likely to benefit from an endocrine treatment and treatment of said patient

### 5) A non-limiting example

A breast cancer patient can present symptoms or signs such as a palpable lump/tumor, secretion from the mamilla or skin deformities. A proportion of breast cancers, generally without symptoms, are also detected by screening mammography.

Following the establishment of a breast cancer diagnosis in a patient, a biopsy of the suspected tumor tissue is performed. A biopsy is the removal of a selected physical piece of tissue from the suspected tumor.

Further, for the provision of a reference sample showing a weak CRABP2 staining, a biopsy is taken from a tissue having low CRABP2 expression, e.g. a biopsy comprising myocytes from skeletal muscle. And for the provision of a reference sample showing a high CRABP2 staining, a biopsy is taken from a tissue having high CRABP2 expression, e.g. a biopsy of tissue showing a staining score of 3 in Table 1 above, such as biopsy comprising surface epithelial cells from the vagina.

The biopsy material is fixated in buffered formalin and histo-processed in order to obtain a thin section (4 µm) of the of the biopsy material.

Immunohistochemistry is performed as described in Examples, section 3. One or more sample sections from each biopsy are mounted on glass slides that are incubated for 45 min in 60 °C, de-paraffinized in xylene (2 x 15 min) and hydrated in graded alcohols. For antigen retrieval, slides are immersed in TRS (Target Retrieval Solution, pH 6.0, DakoCytomation) and boiled for 4 min at 125 °C in a Decloaking chamber® (Biocare Medical). Slides are placed in the Autostainer® (DakoCytomation) and endogenous peroxidase is initially blocked with H₂O₂ (DakoCytomation). The reason for mounting multiple biopsy sample section may be to increase the accuracy of the results.

A primary CRABP2 specific antibody is added to the slides and incubated for 30 min in room temperature, followed by a 30 min incubation in room temperature with the labeled secondary antibody; e.g. goat-anti-rabbit peroxidase conjugated Envision®. To detect the secondary antibody, diaminobenzidine (DakoCytomation) is used as chromogen, contrasted with a Harris hematoxylin (Sigma-Aldrich) counterstaining. Between all steps, slides are rinsed in wash buffer (DakoCytomation). The slides are then mounted with Pertex® (Histolab).

As a tool to validate the staining procedure, two control cell-lines may be used; e.g. one slide with cells expressing CRABP2 (positive cell line) and one slide with cells without CRABP2 expression (negative cell line). The skilled artisan understands how to provide such cell lines, for example guided by the disclosure of Rhodes et al. (2006) The biomedical scientist, p 515-520. The control-line slides may be simultaneously stained in the same procedure as the breast cancer slides, i.e. incubated with the same primary and secondary antibodies.

For example, the breast cancer tumor slides, the staining reference slides (derived from the reference samples), and optionally, the slides with control cell-lines, may be scanned in a light microscope using a ScanScope T2 automated slide scanning system (Aperio Technologies) at x20 magnification.

If control cell-lines are used, these are inspected to validate the staining procedure. If the cell-lines display staining results outside acceptable criteria, e.g. staining artifacts recognized by the skilled artisan, the staining of the biopsy samples is considered as invalid and the whole staining procedure is repeated with new slides. If the positive and negative cell-lines display strong staining intensity and no staining intensity, respectively, the staining is considered as valid.

The stained biopsy slides are evaluated manually by visual inspection in accordance to standards used in clinical histo-pathological diagnostics, and the immunoreactivity of the breast cancer slides are graded as described in Examples, section 4a:
i) The nuclear fraction (nf) is determined, corresponding to the percentage of cells in the sample that exhibits a positive staining in the nucleus. The nuclear fraction of the cell sample is subjectively classified as < 2 %, 2-25 %, 26-75 % or > 75 %.
ii) The nuclear intensity (ni) is determined, corresponding to the over-all staining intensity of the nf-positive cells. The outcome is subjectively classified as: absent (0) = no immunoreactivity, weak (1) = faint immunoreactivity, moderate (2) = medium immunoreactivity or strong (3) = distinct and strong immunoreactivity.

The person performing the evaluation and grading is aided by visual inspection of the stained reference slides having high and low amount of CRABP2 protein expression, respectively.

The breast tumor samples are then categorized as CRABP2 negative, corresponding to nf < 25 % and ni = 0-1, or CRABP2 positive, corresponding to nf >=25% and or ni= 2-3. A CRABP2 positive sample value is indicative of a positive response to an endocrine treatment, such as a tamoxifen treatment. The final decision to apply an endocrine treatment may depend on several parameters. However, a conclusion that the patient is CRABP2 positive is in favor of a decision to treat the patient with an endocrine treatment, especially a tamoxifen treatment.

## Claims

1. Method for determining whether a mammalian subject having a breast cancer is likely to benefit from an endocrine treatment, comprising the steps of:
a) providing a sample earlier obtained from said subject;
b) evaluating the amount of CRABP2 protein present in at least part of said sample, and determining a sample value corresponding to said amount;
c) comparing the sample value obtained in step b) with a reference value; and, if said sample value is higher than said reference value,
d) concluding that the subject is likely to benefit from an endocrine treatment.

2. Method of treatment of a breast cancer in a subject in need thereof, comprising the steps of:
a) providing a sample from said subject;
b) evaluating the amount of CRABP2 protein present in at least part of said sample, and determining a sample value corresponding to said amount;
c) comparing the sample value obtained in step b) with a reference value; and, if said sample value is higher than said reference value,
d) treating said subject with an endocrine treatment regimen.

3. Method according to any one of claims 1 and 2, wherein said breast cancer is ER negative or PR negative.

4. Method according to claim 3, wherein said breast cancer is ER negative.

5. Method for treatment of an ER negative or PR negative breast cancer in a subject, comprising
a) providing a sample from said subject;
b) evaluating the amount of CRABP2 protein present in at least part of said sample, and determining a sample value corresponding to said amount;
c) comparing the sample value obtained in step b) with a reference value; and, if said sample value is lower than, or equal to, said reference value,
d) treating said subject with a non-endocrine treatment regimen.

6. Method according to claim 5, wherein said non-endocrine treatment is a chemotherapy.

7. Method according to claim 5, wherein said non-endocrine treatment is a radiation therapy.

8. Method according to any one of claims 1-4, wherein said endocrine treatment is selected from a SERM treatment, an aromatase inhibitor treatment, and a steroidal estrogen receptor antagonist treatment.

9. Method according to any one of claims 1-4 and 8, wherein said endocrine treatment is a SERM treatment and said SERM is selected from toremifene, raloxifene, droloxifene, arzoxifene and tamoxifene.

10. Method according to any one of claims 1-4 and 8-9, wherein said endocrine treatment is tamoxifen treatment

11. Method according to any preceding claim, in which the sample is a body fluid sample.

12. Method according to claim 11, in which the body fluid is selected from the group consisting of blood, plasma, serum, cerebral fluid, urine and exudate.

13. Method according to any one of claims 1-10, wherein said sample is a tissue sample.

14. Method according to claim 13, wherein said tissue sample is a breast cancer tissue sample.

15. Method according to claim 14, wherein the evaluation of step b) is limited to evaluating the amount of CRABP2 protein expression in the nucleus of tumor cells of said tissue sample.

16. Method according to any one of claims 1-10, wherein said sample is a cytology sample.

17. Method according to any preceding claim, wherein said subject is a human female.

18. Method according any preceding claim, wherein said subject is a premenopausal female.

19. Method according to any preceding claim, wherein said reference value is a predetermined value corresponding to the amount of CRABP2 protein expression in a reference sample.

20. Method according to any preceding claim, wherein said reference value is a nuclear fraction of 25 % CRABP2 protein positive cells, or higher.

21. Method according to any preceding claim, wherein said reference value is a nuclear fraction of 50 % CRABP2 protein positive cells, or higher.

22. Method according to any one of claims 1-19, wherein said reference value is a weak nuclear intensity of CRABP2 protein expression, or higher.

23. Method according to any one of claims 1-19 and 22, wherein said reference value is a moderate nuclear intensity of CRABP2 protein expression, or higher.

24. Method according to any one of claims 1-19, wherein said reference value is a nuclear fraction of 25 % CRABP2 protein or higher or a weak nuclear intensity of CRABP2 protein expression or higher.

25. Method according to any preceding claim, wherein the amino acid sequence of the CRABP2 protein comprises a sequence selected from:
i) SEQ ID NO:1; and
ii) a sequence which is at least 85 % identical to SEQ ID NO:1.

26. Method according to any preceding claim, wherein the amino acid sequence of the CRABP2 protein comprises a sequence selected from:
i) SEQ ID NO:2; and
ii) a sequence which is at least 85 % identical to SEQ ID NO:2.

27. Method according to any preceding claim, wherein step b) comprises:
b1) applying to the sample a quantifiable affinity ligand capable of selective interaction with the CRABP2 protein to be quantified, said application being performed under conditions that enable binding of the affinity ligand to any CRABP2 protein present in the sample;
b2) removing non-bound affinity ligand; and
b3) quantifying any affinity ligand remaining in association with the sample.

28. Method according to claim 27, wherein the quantifiable affinity ligand is selected from the group consisting of antibodies, fragments thereof and derivatives thereof.

29. Method according to claim 27, wherein the quantifiable affinity ligand is a protein ligand derived from a scaffold selected from the group consisting of staphylococcal protein A and domains thereof, lipocalins, ankyrin repeat domains, cellulose binding domains, γ crystallines, green fluorescent protein, human cytotoxic T lymphocyte-associated antigen 4, protease inhibitors, PDZ domains, peptide aptamers, staphylococcal nuclease, tendamistats, fibronectin type III domain and zinc fingers.

30. Method according to claim 27, wherein the quantifiable affinity ligand is an oligonucleotide molecule.

31. Method according to any one of claims 27-30, wherein the quantifiable affinity ligand comprises a label selected from the group consisting of fluorescent dyes and metals, chromophoric dyes, chemiluminescent compounds and bioluminescent proteins, enzymes, radioisotopes, particles and quantum dots.

32. Method according to any one of claims 27-31, in which said quantifiable affinity ligand is detected using a secondary affinity ligand capable of recognizing the quantifiable affinity ligand.

33. Method according to claim 32, in which said secondary affinity ligand capable of recognizing the quantifiable affinity ligand comprises a label selected from the group consisting of fluorescent dyes and metals, chromophoric dyes, chemiluminescent compounds and bioluminescent proteins, enzymes, radioisotopes and particles.

34. Kit for carrying out the method according to any preceding claim, which comprises
a) a quantifiable affinity ligand capable of selective interaction with an CRABP2 protein; and
b) reagents necessary for quantifying the amount of the affinity ligand.

35. Kit according to claim 34, in which the quantifiable affinity ligand is selected from the group consisting of antibodies, fragments thereof and derivatives thereof.

36. Kit according to claim 34, in which the quantifiable affinity ligand is a protein ligand derived from a scaffold selected from the group consisting of staphylococcal protein A and domains thereof, lipocalins, ankyrin repeat domains, cellulose binding domains, γ crystallines, green fluorescent protein, human cytotoxic T lymphocyte-associated antigen 4, protease inhibitors, PDZ domains, peptide aptamers, staphylococcal nuclease, tendamistats, fibronectin type III domain and zinc fingers.

37. Kit according to claim 34, wherein the quantifiable affinity ligand is an oligonucleotide molecule.

38. Kit according to any one of claims 34-37, in which the detectable affinity ligand comprises a label selected from the group consisting of fluorescent dyes and metals, chromophoric dyes, chemiluminescent compounds and bioluminescent proteins, enzymes, radioisotopes, particles and quantum dots.

39. Kit according to any one of claims 34-37, in which said reagents necessary for quantifying the amount of the affinity ligand comprise a secondary affinity ligand capable of recognizing the quantifiable affinity ligand.

40. Kit according to claim 39, in which said secondary affinity ligand capable of recognizing the quantifiable affinity ligand comprises a label selected from the group consisting of fluorescent dyes or metals, chromophoric dyes, chemiluminescent compounds and bioluminescent proteins, enzymes, radioisotopes and particles.

41. Kit according to any one of claims 34-40, further comprising a reference sample for provision of the reference value.

42. Kit according to claim 41, wherein the reference sample contains an amount of CRABP2 protein corresponding to a nuclear fraction of 25 % CRABP2 positive cells or higher.

43. Kit according to claim 41 or 42, wherein the reference sample contains an amount of CRABP2 protein corresponding to a nuclear fraction of 50 % CRABP2 positive cells or higher.

44. Kit according to claim 41, wherein the reference sample contains an amount of CRABP2 protein corresponding to a weak nuclear intensity of CRABP2 protein expression or higher.

45. Kit according to claim 41 or 44, wherein the reference sample contains an amount of CRABP2 protein corresponding to a moderate nuclear intensity of CRABP2 protein expression or higher.

46. Kit according to claim 41, wherein the reference sample contains an amount of CRABP2 protein corresponding to a nuclear fraction of 25 %, or higher, and a weak nuclear intensity, or higher.

47. Kit according to any one of claims 41-46, wherein the reference sample is a tissue sample.

48. Use of a CRABP2 protein as an endocrine treatment indicator for a mammalian subject having a breast cancer.

49. Use of a CRABP2 protein, or an antigenically active fragment thereof, in the manufacture of an endocrine treatment indicator for a mammalian subject having a breast cancer.

50. Use according any one of claims 48-49, wherein the amino acid sequence of the CRABP2 protein comprises a sequence selected from:
i) SEQ ID NO:1; and
ii) a sequence which is at least 85 % identical to SEQ ID NO:1.

51. Affinity ligand capable of selective interaction with an CRABP2 protein, which is an antibody or a fragment or a derivative thereof.

52. Affinity ligand according to claim 51, which is obtainable by a process comprising a step of immunizing an animal with a protein whose amino acid sequence comprises the sequence SEQ ID NO:1.

53. Affinity ligand according to any one of claims 51 and 52 for use as an endocrine treatment indicator for a mammalian subject having a breast cancer.

54. Use of an affinity ligand according to any one of claims 51 and 52 as an endocrine treatment indicator for a mammalian subject having a breast cancer.

55. Use of an affinity ligand according to any one of claims 51 and 52 in the manufacture of an endocrine treatment indicator for a mammalian subject having a breast cancer.

56. Endocrine treatment product for use in the treatment of a mammalian subject having a breast cancer, wherein said subject is diagnosed as hormone receptor negative and CRABP2 protein positive.

57. Use of an endocrine treatment product in the manufacture of a medicament for treatment of a mammalian subject having a breast cancer, wherein said subject is diagnosed as hormone receptor negative and CRABP2 protein positive.
